# EUROPEAN PATENT SPECIFICATION

(11) **EP 0 468 527 B1**
(45) Date of publication and mention of the grant of the patent: **21.04.1999**
(21) Application number: 91112620.9
(22) Date of filing: 26.07.1991
(51) Int. Cl.: C07K 14/18, A61K 39/29, G01N 33/569

(54) **Synthetic peptides specific for the detection of antibodies to HCV, diagnosis of HCV infection and prevention thereof as vaccines**
Synthetische Peptide, spezifisch zum Nachweis von gegen HCV gerichteten Antikörpern zur Diagnose der HCV-Infektion und zum Vorkommen davon als Impfstoffe
Peptides synthétiques, spécifiques pour le dépistage d'anticorps contre HCV, pour la diagnose de l'infection causée par HCV et sa prévention en tant que vaccins

(30) Priority: 26.07.1990 US 558799; 07.02.1991 US 651735; 11.03.1991 US 667275; 24.06.1991 US 719819
(43) Date of publication of application: 29.01.1992
(73) Proprietor: United Biomedical, Inc., Hauppauge, New York 11788 (US)
(72) Inventor: Chang Yi Wang, Great Neck, N.Y.11021 (US); Hosein, Barbara, New York, N.Y.10021 (US)
(74) Representative: Hansmann, Axel, Dipl.-Wirtsch.-Ing.

(56) References cited:
- EP-A- 0 318 216
- EP-A- 0 388 232
- EP-A- 0 442 394
- SCIENCE. vol. 244, 21 April 1989, LANCASTER, PA US pages 362 - 364; G KUO ET AL.: 'an assay for circulating antibodies to a major etiologic virus of human non-a, non-b hepatitis'
- SCIENCE. vol. 244, 21 April 1989, LANCASTER, PA US pages 359 - 362; QL CHOO ET AL.: 'isolation of a cdna clone derived from a blood-borne non-a, non-b viral hepatitis genome'
- NUCLEIC ACIDS RESEARCH. vol. 17, no. 24, 1989, ARLINGTON, VIRGINIA US pages 10367 - 10372; Y KUBO ET AL.: 'a cdna fragment of hepatitis c virus isolated from an implicated donor of post-transfusion non-a, non-b hepatitis in japan'
- JOURNAL OF IMMUNOLOGICAL METHODS. vol. 124, no. 1, 1989, NEW YORK US pages 53 - 61; J P TAM AND F ZAVALA: 'multiple antigen peptide. A novel approach to increase detection sensitivity of synthetic peptides in solid-phase immunoassays'

## Description

### INTRODUCTION

The present invention relates to peptides specific for the diagnosis and prevention of hepatitis C virus (HCV) infection, or non-A non-B hepatitis (NANBH). More particularly, the present invention is directed to synthetic peptides which are specific for the detection of antibodies to HCV in body fluids and immunoassays using the same. The invention also includes the use of the synthetic peptides in compositions as antigens for eliciting the production of monoclonal and polyclonal antibodies against HCV and as immunogens in vaccines for the prevention of NANBH or HCV infection.

In recent years, non-A, non-B hepatitis (NANBH) has become the most common form of post-transfusion hepatitis. Studies involving the experimental inoculation of chimpanzees provided evidence that the infectious agent was a lipid-containing virus resembling members of the Togaviridae family.

Recently, this etiological agent, termed hepatitis C virus (HCV) has been shown to be an RNA virus with a genome size of ∼ 10 kilobases encoding a single polyprotein which can be further processed into several structural and nonstructural proteins (1-4). Additional computer-assisted protein analysis demonstrates that HCV shares sequence similarity with the polyproteins of animal pestiviruses and flaviviruses as well as members of two plant virus supergroups (5).

More recently, a number of reports have led to an increasingly coherent understanding of the function of various regions of the virus and of the relationships among genomic fragments isolated from variants or closely related viruses.

A summary of the HCV structure, beginning at the N terminus of the virus, follows. The HCV comprises a structural protein region and nonstructural (NS) protein regions. The structural protein region is further divided into capsid and envelop proteins. The NS protein regions are further divided into NS-1 to NS-5 regions (3).

The postulated capsid region (AA1-AA120) has been shown to contain highly immunoreactive conserved epitopes with enhanced sensitivity in the detection of hepatitis C infection (6-8). The region appears to consist of two segments of equal length (AA1-61, AA62-AA120), which are homologous to one another, perhaps as a result of a gene duplication, and are also homologous to the N terminal core region of yellow fever virus (9), also a flavivirus (Table 1A). Both halves, as represented by peptides VIIIE (AA2-AA62) and IXD (AA65-AA119), disclosed in application serial No. 558,799, have been shown to be immunoreactive. A genomic fragment of a NANBH virus cloned by Arima et al. (10), designated clone 2, contains a Gly-Pro-Arg-Leu-Gly sequence identical to residues 39-43 in peptide VIIIE (Table 1B), placing this clone 2 fragment in the putative core region of a related virus. Two other sequences from NANBH viruses, cloned by Reyes et al. (11) and by Arima et al. (clone 1) (12), show sequence similarities with the capsid region of yellow fever virus (Table 1C). Thus, there appears to be a number of related viruses, all of which have highly immunogenic capsid regions, as evidenced by the ease of cloning. Variants of hepatitis C (J, J-1, J-4) are also highly conserved in this region (2-4), so the other clones mentioned by Arima et al. may be from different viruses, rather than from variants of HCV.

Mishiro and colleagues have isolated a cDNA clone from the plasma of a chimpanzee infected with NANBHV which codes for a host cellular sequence bearing an epitope which is reactive with sera from individuals who are PCR positive for HCV (13). The sequence of the immunoreactive peptide (GOR epitope) is not encoded by HCV and was reported not to resemble a published sequence of HCV spanning three-quarters of the genome (1) or the 5'-terminal sequence of HCV (2) covering the upstream quarter of the genome. However, inspection of the GOR epitope sequence revealed 47% homology with an N-terminal fragment covered by peptide VIIIE described in UBI Applications Serial No. 558,799. Lesser degrees of homology were obtained from comparison with the N-terminus of the yellow fever virus capsid protein (33%) (9) and the protein segment corresponding to clone 1 of Arima et al. (37.5%) (12) (See Table 1D).

The presence of antibodies which are cross-reactive with the GOR epitope sequence in HCV infected individuals may be explained by structural similarity of the GOR epitope with the corresponding region of the HCV capsid protein. Compared with anti-C100, antibodies to the C100 region, previously identified by Houghton et al.; antibodies to peptide VIIIE share the following characteristics with anti-GOR: they both are present in some but not all anti-C100 positive sera; they can be detected in anti-C100 negative sera from both acute and chronic NANBH patients; they appear earlier than anti-C100 in the seroconversion series; they are detected in more seroconversion panels than anti-C100 (13); and they are present in 1-2% of normal controls and 15-20% of HBsAg positive individuals. Early NANBH assays reported to react with host-determinant cytoplasmic antigens may in fact have detected anti-HCV capsid protein cross-reactivity.

The postulated envelope (env) region consists of amino acids 120 to 400. The env glycoproteins of flaviviruses are key targets for immunization because the env region is a major antigen of free viral particles and plays a central role in flavivirus biology. The env region mediates binding to cell receptors and probably facilitates fusion to membranes. It also induces protective immune responses after vaccination or natural infection with a flavivirus (14,15) and stimulates cell-mediated immunity (16). The type-specific epitopes on env are the ones most closely associated with protective immune responses to flaviviruses (17-19). There are a number of hypervariable regions in the HCV env region, based on a comparison of US and Japanese strains (2), which may indicate epitopes for strain specific reactivity.

The non-structural protein NS-1, in addition to the small M protein of the envelope, has been shown to contribute to protective immunity in dengue fever (20,21). Inspection of sequences and hydrophobicity profiles shows that the HCV NS-1 region contains two similar domains (Table 1E). A dominant motif in, this region is cysteine pairs separated by five or more amino acids.

The NS-2 region is of unknown function and little has been reported on its characteristics.

By analogy with yellow fever virus, the HCV NS-3 region may contain protease activity required for viral replication (22). A trypsin-like serine protease active site has been localized in yellow fever virus by means of site-directed mutagenesis of NS-3 to a catalytic triad consisting of His-53, Asp-77 and Ser-138. The corresponding region in HCV is the N-terminal third of NS-3, with the critical residues being His-1103, Asp-1127 and Ser-1188. The remainder of the HCV NS-3 region consists of a region which shows immunoreactivity. This region appears to consist of three subregions homologous to one another (Table 1F) and these subregions bear a distant relationship to the repeated segments of the NS-1 region.

The most widely studied region to date is the NS-4 nonstructural region. Although its function is unknown, it contains highly immunoreactive regions, primarily in the region designated as C100 by Houghton et al. (1), which became the basis for a HCV diagnostic test using recombinant technology. A high degree of structural homology is observed between part of the C100 HCV sequence with a corresponding region in the yellow fever virus (Table 1G). While this region detects antibody to the virus primarily responsible for NANBH (23), experimentally it has been shown in prior United Biomedical Inc.'s application Serial No. 558,799 and numerous recent reports that there are shortcomings in both sensitivity and specificity in the tests relying on the C100 polypeptide as an antigen. However, synthetic peptides from the NS-4 region described in prior application Serial No. 558,799 overcome the problem of non-specific reactivity.

The nonstructural region proximal to the C terminus of HCV is NS-5, the site of polymerase (pol) activity. The Gly-Asp-Asp sequence in this region is conserved across many viruses(11). Maeno et al. have isolated a clone corresponding to a sequence upstream of the pol site in the NS-5 region which is immunoreactive and which reacts specifically with sera from patients in the chronic phase of NANBH(24).

Through an extensive series of experiments involving serological validation using select specimens chosen from the screening of thousands of sera with hundreds of carefully designed synthetic peptides, we have further characterized the capsid protein related immunoreactive peptides and have identified additional immunoreactive epitopes contained within the envelope, NS-1, NS-2, NS-3, and NS-5 protein regions.

Synthetic peptides have been increasingly used to map antigenic or immunogenic sites on the surface of proteins, an approach recently termed "site-directed-serology". We, at United Biomedical, have taken this approach to identify and characterize highly antigenic epitopes on the envelope and core proteins of HIV and to develop sensitive and specific immunoassays for the detection of antibodies to HIV (previously designated HTLV-III) (25-27). See U.S. Patent 4,735,896, issued April 5, 1988 and (U.S. Patent 4,879,212 issued Nov. 7, 1989, the contents of which are, hereby, fully incorporated by reference (28,29). Subsequently, a series of finely mapped and well-characterized HTLV-I/II related synthetic peptides were employed in the development of synthetic peptide-based diagnostic assays for the detection of HTLV-I/II antibodies in infected individuals (30,31). See also U.S. Patent 4,833,071 issued May 23, 1989, U.S.S.N. 07/297,635 filed January 13, 1989 and USSN 07/469,294 filed January 24, 1990. These assays have provided superior sensitivity, excellent specificity, and, in certain cases, an unmatched capability to differentiate infections between two closely related viruses, thus overcoming many of the existing problems associated with biologically-derived tests based on either viral lysates or recombinant DNA-derived proteins.

From EP-A-0 388 232 a recombinant polynucleotide comprising sequences derived from HCV cDNA is known including the application of these sequences and polypeptides in immunoassays, probe diagnostics, anti-HCV antibody production, PCR technology and recombinant DNA technology. Disclosed are also immunogenic polypeptides encoded within clones containing HCV cDNA, methods for purifying an immunogenic HCV polypeptide and antisense polynucleotides derived from HCV cDNA.

It is, therefore, an objective of the present invention to employ the identified and characterized immunoreactive HCV peptides in the development of a detection or diagnostic procedure to identify and monitor HCV infection.

A further objective is to chemically synthesize a test reagent which can then be used to detect the presence of antibodies to HCV in body fluids and to diagnose NANBH.

Another objective is to develop a vaccine which, when introduced into healthy mammals, including humans, will stimulate production of efficacious antibodies to HCV, thereby providing protection against HCV infection.

A further objective is to provide a synthetic immunogen which can be used in mammals for the development of monoclonal and polyclonal antibodies to HCV.

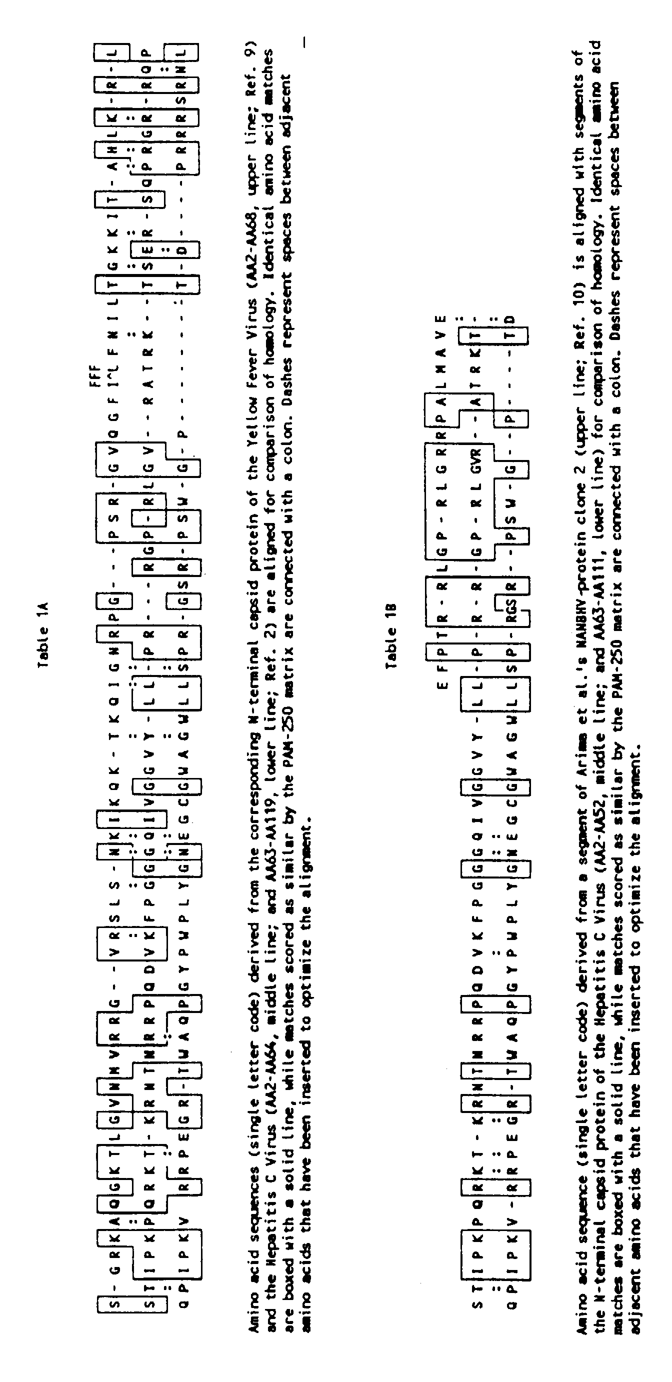

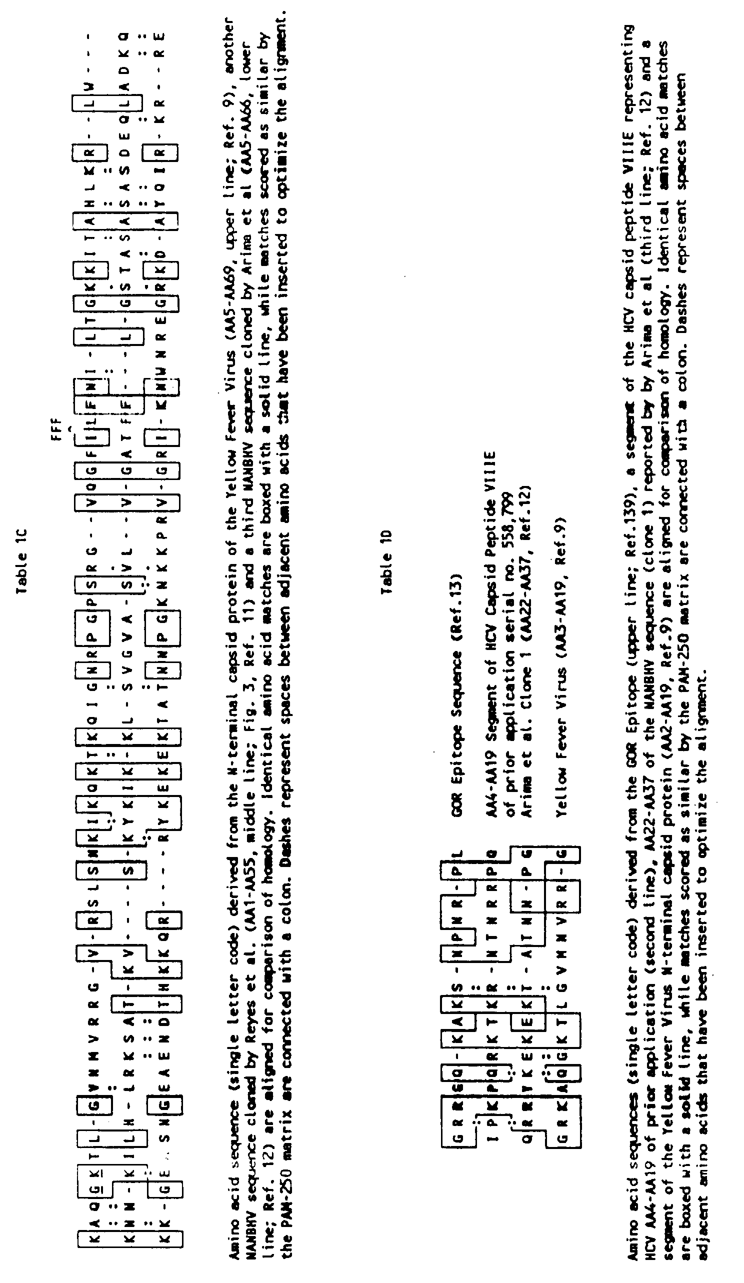

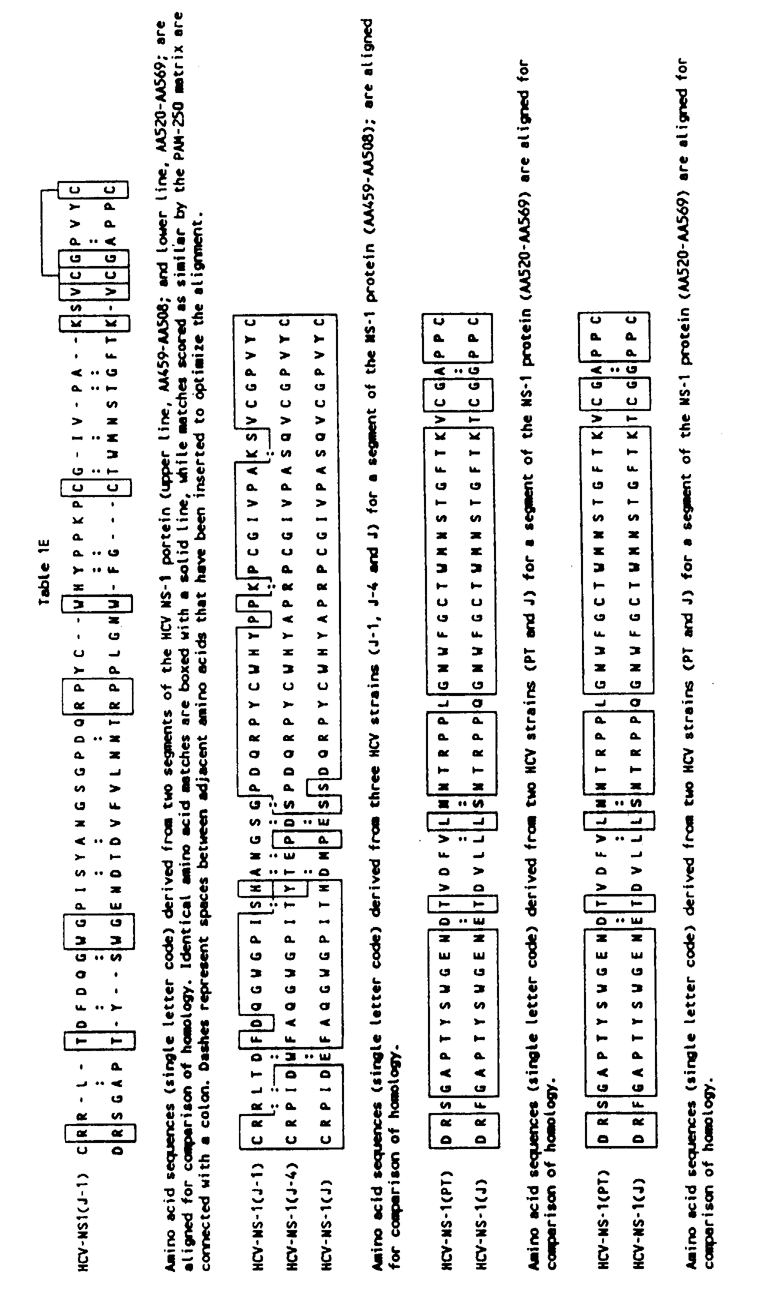

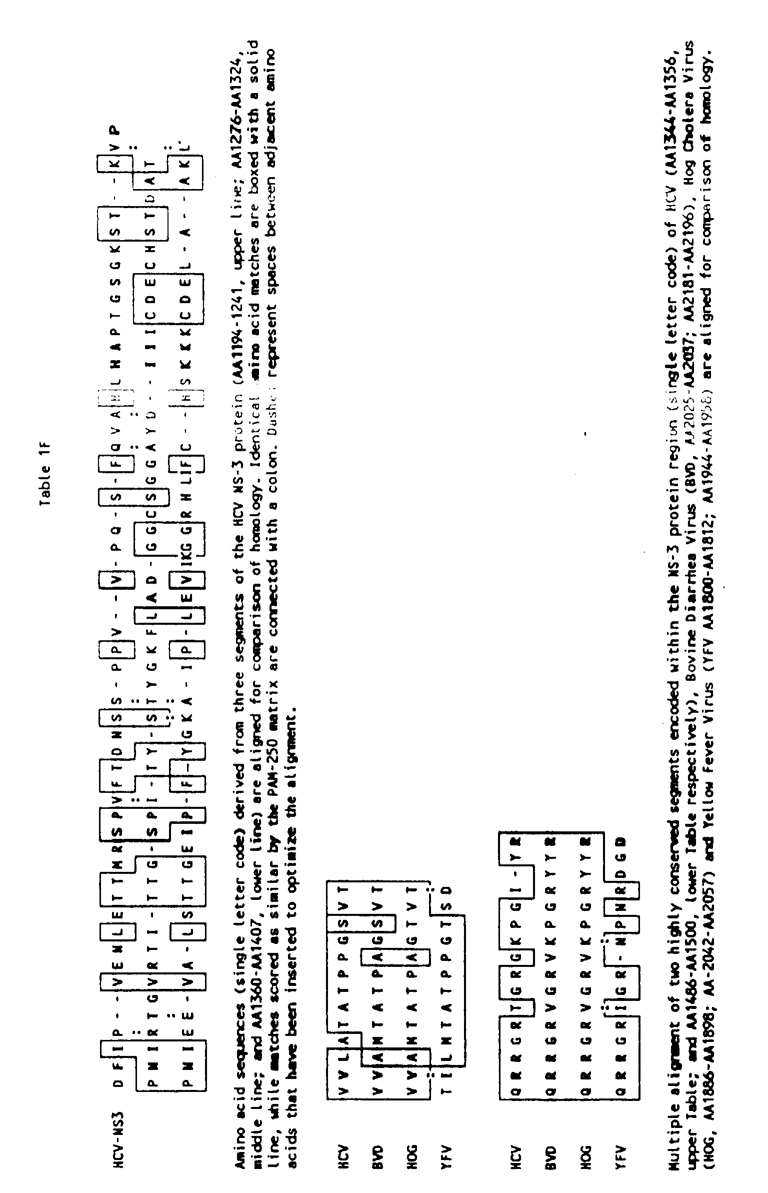

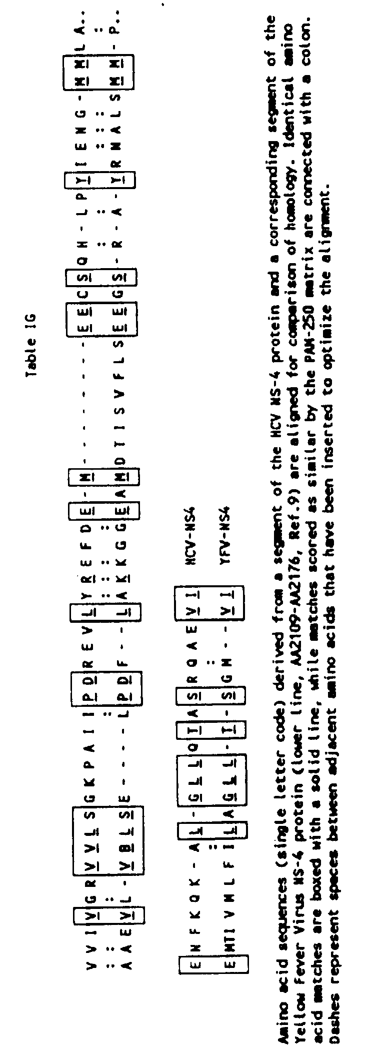

### REFERENCES

1. Houghton M, Choo Q-L, Kuo G: NANBV diagnostics and vaccines. EPO 0318216A1 (1989).
2. Okamoto H, Okada S, Sugiyama S, Yotsumoto S, Tanaka T, Yoshizawa H, Tsuda F, Miyakawa Y, Mayumi M: The 5' terminal sequence of the hepatitis C virus genome. Jpn. J. Exp. Med. 60:167 (1990).
3. Houghton M, Choo Q-L, Kuo G: NANBH diagnostics and vaccines. EPO 0388232A1 (1990).
4. Kato N, Hijikata M, Ootsuyama Y, et al: Molecular cloning of the human hepatitis C virus genome from Japanese patients with non-A, non-B hepatitis. Proc. Natl. Acad. Sci. USA 87:9524 (1990).
5. Miller RH, Purcell RH: Hepatitis C virus shares amino acid sequence similarity with pestiviruses and flaviviruses as well as members of two plant virus supergroups. Proc. Natl. Acad. Sci. USA 87:2057 (1990).
6. Hosein B, Fang CT, Zhang ML, et al: Improved serodiagnosis of hepatitis C virus infection with synthetic peptide antigen from capsid protein. Proc. Natl. Acad. Sci. USA 88:3647 (1991).
7. UBI HCV EIA Product Insert. (1990).
8. Okamoto H, Munekata E, Tsuda F, et al: Enzyme-linked immunosorbent assay for antibodies against the capsid protein of hepatitis C virus with a synthetic oligopeptide. Jap. J. Exp. Med. 60:223 (1990).
9. Rice CM, Lencheo EM, Eddy SR, et al: Nucleotide sequence of yellow fever virus: Implications for flavivirus gene expression and evolution. Science 229:726 (1985).
10. Arima T, Takamizawa A, Mori C, et al: A lambda gt11-cDNA clone specific for chronic hepatitis C generated from pooled serum presumably infected by hepatitis C virus. Gastroenterologia Japonica 24:545 (1989).
11. Reyes GR, Purdy MA, Kim J, et al: Isolation of a cDNA from the virus responsible for enterically transmitted non-A, non-B hepatitis. Science 247:1335 (1990).
12. Arima T, Nagashima H, Murakami S, et al: Cloning of a cDNA associated with acute and chronic hepatitis C infection generated from patients serum RNA. Gastroenterologia Japonica 24:540 (1989).
13. Mishiro S, Hoshi Y, Takeda K, et al; Non-A, non-B hepatitis specific antibodies directed at host-derived epitope: Implication for an autoimmune process. Lancet 336:1400 (1990).
14. Brinton MA: in The Togaviridae and Flaviviridae, ed. Schlesinger S and Schlesinger MJ. Plenum Press, NY pp. 327-374 (1986).
15. Mandl CW, Guirakhoo F, Holzmann H, Heinz FX, Kunz C: Antigenic structure of the flavivirus envelope protein E at the molecular level, using tick-borne encephalitis virus as a model. J. Virol. 63:564 (1989).
16. Bray M, Falgout B, Zhao B, et al: in Vaccines '89. Modern Approaches to New Vaccines Including Prevention of AIDS, ed Lerner RA, Ginsberg H, Chanock RM and Brown F. Cold Spring Harbor Laboratory, NY, pp357-362 (1989).
17. Roehrig JT, Hunt AR, Johnson J, Mathews JH: ibid. pp347-350 (1989).
18. Rothman AL, Kurane J, Ennis FA: ibid. pp363-366 (1989).
19. Roehrig JT: in The Togaviridae and Flaviviradae, ed Schlesinger S and Schlesinger MJ. Plenum Press NY, pp251-278 (1986).
20. Bray M, Meu R, Lai CJ: Meeting on Modern Approaches to New Vaccines Including Prevention of AIDS. Cold Spring Harbor Laboratory, Sept 12-16, 1990. Abst 70.
21. Falgout B, Bray M, Schlesinger JJ, Lai CJ: Immunization of mice with recombinant vaccinia virus expressing authentic dengue virus nonstructural protein NS1 protects against lethal dengue virus encephalitis. J. Virol. 64:4356 (1990).
22. Chambers TJ, Weir RC, Grakoui A, et al: Evidence that the N-terminal domain of nonstructural protein NS-3 from yellow fever virus is a serine protease responsible for site-specific cleavages in the viral polyprotein. Proc. Natl. Acad. Sci. USA 87:8898 (1990).
23. Kuo G., Choo Q-L, Alter HJ, et al: An assay for circulating antibodies to a major etiologic virus of human non-A, non-B hepatitis: Science 244:362 (1989).
24. Maeno M, Kaminaka K, Sugimoto H, et al: A cDNA clone closely associated with non-A, non-B hepatitis. Nucleic Acids Res. 18:2685 (1990).
25. Wang CY: Synthetic-peptide-based immunodiagnosis of retrovirus infections: Current status and future prospects. In: Synthetic Peptides in Biotechnology, ed. Mizrahi A, Advances in Biotechnological Processes, 10:131 (1988).
26. Wang JG, Steel S, Wisniewolski R, Wang CY: Detection of antibodies to HTLV-III using a synthetic peptide of 21 amino acid residues corresponding to a highly antigenic segment of gp41 envelope protein. Proc. Natl. Acad. Sci. USA 83:6159 (1986).
27. Wang CY: European Patent Application Publication: EPO 0328403 (1989). Synthetic peptides related to the HIV-gp120 env protein, and their use.
28. Wang CY, Wang JG: U.S. Patent 4879212 (1989). Peptide composition and method for the detection of antibodies to HTLV-III.
29. Wang CY, Wang JG: U.S. Patent 4735896 (1988). Synthetic peptide and process of using same for the detection and diagnosis of AIDS and pre-AIDS conditions.
30. Wang CY, Wang JG, Walters DW: U.S. Patent 4833071 (1989). Peptide composition as antigen for detection of antibodies to HTLV-I, as a vaccine for ATL, and methods therefore.
31. Wang CY: U.S.S.N. 07/297635. Synthetic peptide compositions with immunoreactivities to antibodies to HTLV.

### BRIEF DESCRIPTION OF THE INVENTION

According to the present invention, a series of synthetic peptides representing immunoreactive regions of the postulated envelope protein and nonstructural proteins NS-1, NS-2, NS-3 and NS-5 of the hepatitis C virus (HCV), each arranged in a specific sequence, has been identified and made by solid phase peptide synthesis. These peptides have been found to be useful for the detection of antibodies to HCV in sera and body fluids and for the diagnosis of non-A, non-B hepatitis (NANBH). Because of their immunoreactivity, it is expected that these peptides are also useful in stimulating production of antibodies to HCV in healthy mammals such as Balb/C mice, and in a vaccine composition to prevent HCV or NANBHV infection.

According to the present invention, a peptide composition useful for the detection of antibodies to HCV and diagnosis of NANBH comprises a peptide from the envelope, NS-1, NS-2, NS-3 and NS-5 regions of the HCV represented by the following sequences:

Phe-Thr-Phe-Ser-Pro-Arg-Arg-His-Trp-Thr-Thr-Gln-Gly-Cys-Asn-
Cys-Ser-Ile-Tyr-Pro-Gly-His-Ile-Thr-Gly-His-Arg-Met-Ala-Trp-
Asp-Met-Met-Met-Asn-Trp-Ser-Pro-Thr-Ala-X
Pep8

Glu-Ile-Leu-Arg-Lys-Ser-Arg-Arg-Phe-Ala-Gln-Ala-Leu-Pro-Val-
Trp-Ala-Arg-Pro-Asp-Tyr-Asn-Pro-Pro-Leu-Val-Glu-Thr-Trp-Lys-
Lys-Pro-Asp-Tyr-Glu-Pro-Pro-Val-Val-His-Gly-Cys-Pro-Leu-Pro-
Pro-Pro-Lys-Ser-Pro-Pro-Val-Pro-Pro-Pro-Arg-Lys-Lys-Arg-Thr-
X
Pep11

Lys-Ala-Thr-Cys-Thr-Ala-Asn-His-Asp-Ser-Pro-Asp-Ala-Glu-Leu-
Ile-Glu-Ala-Asn-Leu-Leu-Trp-Arg-Gln-Glu-Met-Gly-Gly-Asn-Ile-
Thr-Arg-Val-Glu-Ser-Glu-Asn-Lys-Val-Val-Ile-Leu-Asp-Ser-Phe-
Asp-Pro-Leu-Val-Ala-Glu-Glu-Asp-Glu-Arg-X
Pep12

These 3 peptides are in addition to Peptide VIIIE, a peptide from the structural protein region, and Peptide IIH, peptides from the non-structural protein region which have also been found to be reactive and useful for the detection of antibodies to HCV and diagnosis of NANBH.

Peptide VIIIE has the following sequence:

Ser-Thr-Ile-Pro-Lys-Pro-Gln-Arg-Lys-Thy-Lys-Arg-Asn-Thr-Asn-Arg-
Arg-Pro-Gln-Asp-Val-Lys-Phe-Pro-Gly-Gly-Gly-Gln-Ile-Val-Gly-Gly-
Val-Tyr-Leu-Leu-Pro-Arg-Arg-Gly-Pro-Arg-Leu-Gly-Val-Arg-Ala-Thr-
Arg-Lys-Thr-Ser-Glu-Arg-Ser-Gln-Pro-Arg-Gly-Arg-Arg-X,

Peptide IIH has the following sequence: 
Ser-Gly-Lys-Pro-Ala-Ile-Ile-Pro-Asp-Arg-Glu-Val-Leu-Tyr-Arg-Glu-
Phe-Asp-Glu-Met-Glu-Glu-Cys-Ser-Gln-His-Leu-Pro-Tyr-Ile-Glu-Gln-
Gly-Met-Met-Leu-Ala-Glu-Gln-Phe-Lys-Gln-Lys-Ala-Leu-Gly-Leu-X
 wherein X is -OH or -NH₂ and analogues, segments, mixtures, conjugates and polymers thereof.

Further, according to the present invention, the peptides by themselves, or when coupled to a protein or a polymeric carrier of homo or hetero dimers or higher oligomers by the use of homo or hetero functional multivalent cross linking reagents, or when directly synthesized and conjugated to a branching polyvalent lysine resin, can be used to elicit the production of antibodies to HCV in healthy mammals, including humans.

The method comprises introducing an effective amount of the peptide composition containing each of the individual peptides, analogues or segments or a mixture or a combination thereof, or in a polymeric form, into the body of a healthy mammal by intraperitoneal or subcutaneous injection.

Vaccines containing the peptides according to the present invention as the key immunogen may also be prepared as described above or by known methods. It is expected that such vaccine compositions may be useful to prevent HCV infection or NANBH.

### BRIEF DESCRIPTION OF DRAWING

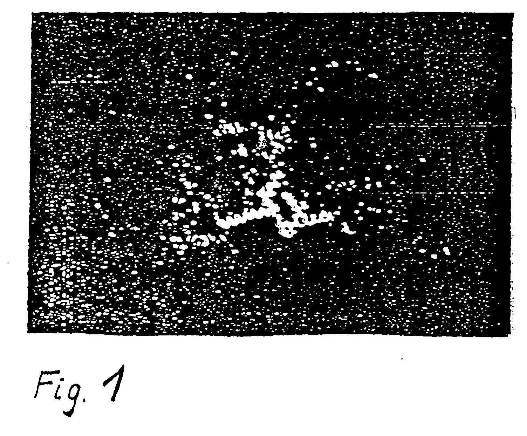

Fig. 1 is a photograph of a computer-generated structure of an octameric peptide immunogen.

### DETAILED DESCRIPTION OF THE INVENTION

In accordance with the present invention, three peptides and their analogues have been selected from the nonstructural regions of HCV and chemically synthesized. These peptides including their analogues are useful for the detection of antibodies to HCV in body fluids, the diagnosis of NANBH, and for the vaccination of healthy mammals by stimulating the production of antibodies to HCV. These peptides are arranged in the following sequences:

Phe-Thr-Phe-Ser-Pro-Arg-Arg-His-Trp-Thr-Thr-Gln-Gly-Cys-Asn-
Cys-Ser-Ile-Tyr-Pro-Gly-His-Ile-Thr-Gly-His-Arg-Met-Ala-Trp-
Asp-Met-Met-Met-Asn-Trp-Ser-Pro-Thr-Ala-X
Pep8

Glu-Ile-Leu-Arg-Lys-Ser-Arg-Arg-Phe-Ala-Gln-Ala-Leu-Pro-Val-
Trp-Ala-Arg-Pro-Asp-Tyr-Asn-Pro-Pro-Leu-Val-Glu-Thr-Trp-Lys-
Lys-Pro-Asp-Tyr-Glu-Pro-Pro-Val-Val-His-Gly-Cys-Pro-Leu-Pro-
Pro-Pro-Lys-Ser-Pro-Pro-Val-Pro-Pro-Pro-Arg-Lys-Lys-Arg-Thr-
X
Pep11

Lys-Ala-Thr-Cys-Thr-Ala-Asn-His-Asp-Ser-Pro-Asp-Ala-Glu-Leu-
Ile-Glu-Ala-Asn-Leu-Leu-Trp-Arg-Gln-Glu-Met-Gly-Gly-Asn-Ile-
Thr-Arg-Val-Glu-Ser-Glu-Asn-Lys-Val-Val-Ile-Leu-Asp-Ser-Phe-
Asp-Pro-Leu-Val-Ala-Glu-Glu-Asp-Glu-Arg-X
Pep12

These 3 peptides are in addition to Peptide VIIIE, a peptide from the structural protein region, and Peptide IIH, peptides from the non-structural protein region which have also been found to be reactive and useful for the detection of antibodies to HCV and diagnosis of NANBH.

Peptide VIIIE has the following sequence:

Ser-Thr-Ile-Pro-Lys-Pro-Gln-Arg-Lys-Thr-Lys-Arg-Asn-Thr-Asn-Arg-
Arg-Pro-Gln-Asp-Val-Lys-Phe-Pro-Gly-Gly-Gly-Gln-Ile-Val-Gly-Gly-
Val-Tyr-Leu-Leu-Pro-Arg-Arg-Gly-Pro-Arg-Leu-Gly-Val-Arg-Ala-Thr-
Arg-Lys-Thr-Ser-Glu-Arg-Ser-Gln-Pro-Arg-Gly-Arg-Arg-X,

Peptide IIH has the following sequence: 
Ser-Gly-Lys-Pro-Ala-Ile-Ile-Pro-Asp-Arg-Glu-Val-Leu-Tyr-Arg-Glu-
Phe-Asp-Glu-Met-Glu-Glu-Cys-Ser-Gln-His-Leu-Pro-Tyr-Ile-Glu-Gln-
Gly-Met-Met-Leu-Ala-Glu-Gln-Phe-Lys-Gln-Lys-Ala-Leu-Gly-Leu-X
 Wherein X is -OH or -NH₂ and analogues, segments, mixtures, conjugates, and polymers thereof.

These peptides may comprise combinations or segments, i.e. longer or shorter peptide chains by having more amino acids added to the terminal amino acids, or by amino acids removed from either terminal end.

These peptides may also comprise analogues to accommodate strain-to-strain variations among different isolates of HCV. HCV is indicated to have frequent mutations. Therefore, it is expected that variant strains, such as PT, J, J-1 and J-4 (1-4) exist. Adjustments for conservative substitutions and selection among the alternatives where non-conservative substitutions are involved, may be made in the prescribed sequences (e.g. see Table 1E for possible amino acid substitutions in the hypervariable regions of the envelope and NS-1 proteins). These analogues of the synthetic peptides may therefore comprise substitutions, insertions and/or deletions of the recited amino acids of the above sequence to accomodate the various strains, as long as the immuno-reativity recognizable by the antibodies to HCV is preserved

These peptides may also comprise conjugates, i.e., they may be coupled to carrier proteins such as bovine serum albumin (BSA) or human serum albumin (HSA). Furthermore, these peptides may comprise polymers, i.e., they may be synthesized on a polymeric resin or in dimeric, tetrameric, octameric and decahexyl forms of the peptide or their analogues, such as a branching octameric lysine resin.

The branchine poly-L-lysine can be Lys₈ Lys₄ Lys₂ Lys, Lys₄ Lys₂ Lys, Lys₂ Lys,Lys; the last Lys can be attached to Y as in Lys₄ Lys₂ Lys-Y wherein Y is -OH, -NH₂ or an amino acid containing no side chain functional group, such as alanine, valine, glycine, etc. Y can be inserted to facilitate synthesis onto the 4-methylbenzylhydrylamine resin. The conjugates and polymers of the peptides are also useful in the present invention.

The amino acid sequences of the polypeptide as described in the invention useful as test reagents for the detection of antibodies to HCV in body fluids and diagnosis of NANBH are selected to correspond to segments of the amino acid sequence of the postulated envelope and non-structural proteins of HCV designated as env, NS-1, NS-2, NS-3 and NS-5 based on amino acid sequence information derived from Houghton et al. (13), Okamoto et al (2) and Kato et al (4).

In selecting regions of the HCV protein for epitope analysis, peptides of about 40 mer size with amino acid sequences covering the complete HCV envelope and non-structural proteins NS-1, NS-2, NS-3 and NS-5 were synthesized. These were tested for their immunoreactivity with special specimens previously selected through the screening of thousands of patient and normal sera for their unique immunoreactivity with HCV. Three peptides from the postulated envelope and nonstructural protein regions NS-1, NS-2, NS-3 and NS-5 designated as pep8, pep11 and pep12 and their analogues were identified to have specific immunoreactivity with the positive HCV sera.

At present, available knowledge of protein structure has not enabled the scientist to predict the amino acid sequences that may represent highly immunogenic epitopes. The usefulness of a peptide as an antigen or immunogen must be empirically determined. We have only been able to identify and characterize immuno-reactive epitopes through an extensive process which we call "serological validation". The following example shows how difficult it is to identify immuno-reactive epitopes.

For example, a clone designated as C33c encoded within the NS-3 region was reported to possess immunoreactivity(3). This clone spans 265 amino acid residues. Assuming a useful peptide must be at least 6 amino acids in length and that the upper limit for synthetic peptides in reasonable yield is 120 residues, the number of possible unique peptides from the C33c regions is 23,028. For the entire HCV genome, the figure is about 260,000.

In addition, we have shown that extraction conditions are critical for the expression of the immunopotency of a peptide (Example 4C), so the number of uniquely extracted peptides from this region is in multiples of 23,028. The possibilities for post-extraction modification, such as pH adjustment (Example 4B) further increase the possible selections to >10⁶. If amino acid substitutions at various positions are taken into consideration, this figure will quickly increase to several millions. In contrast to the HCV core region, in which peptides VIIIE and IXD were the optimal analogues, longer peptides are not preferred over shorter analogues in the NS-3/C33c region. For example, the 42 mer 279B shown on Table 4D has only 3% of the reactivity of the 37 mer peptide 3, designated as 279A in Table 4D. Of 30 peptides spanning the C33c region tested, only one was found to be useful. The antigenic index as referred in Houghton et al (3) did not prove to be a useful guide to epitopes, as the profile for peptide 3 is positive for only 30% of its sequence and negative for the remaining 70%.

The strategy for serological validation also depends on the expected characteristics of the target epitopes. Universal immunodominant epitopes, such as the gp41 transmembrane peptide of HIV-1, may be screened by a single representative serum sample from a patient known to be infected with the virus. Epitopes which are not recognized by all infected individuals, or those for which antibody is produced late or only transiently, and especially epitopes which give rise to neutralizing antibodies, must be screened by large panels of sera. For example, peptide 272B shown in Table 4A was initially tested on a panel of eight sera from HCV infected individuals (Panel 1). Only one sample was definitely positive with an absorbance of 880 mA. Three were weakly reactive (<200 mA) and four were negative.

The identification of the immuno-reactive epitopes is also dependent on the panel of sera used. The more closely the panel represents the population most likely to be seropositive for the desired epitope, the greater the chance that the epitope will be identified. For example, peptides synthesized from the NS-1 region, which were hypothesized to be important for generating neutralizing antibodies, gave only weakly reactive or negative results on screening with a very large number (n > 200) of samples from individuals who were newly infected and/or chronically infected with HCV. However, a panel of 24 samples from asymptomatic individuals from a known hepatitis virus endemic geographical region, Taiwan and mainland China, yielded two samples with absorbances of >2000 mA against multiple NS-1 peptides.

Finally, if the desired purpose of a targeted peptide/epitope is to extend the range of reactivity of an assay comprised of previously identified epitopes, then a large number of samples from individuals at risk of infection but seronegative against known epitopes must be employed for screening. Unfortunately, the most critical samples from clinically proven and documented cases of infection may be available in quantities insufficient for screening purposes. This is another complication/difficulty encountered in serological validation for determining the immunoreactivity of a peptide.

The process of "serological validation" is particularly difficult when the epitopes to be identified elicit antibodies only in a subpopulation of an infected patient group. When such epitopes become targets for identification, special attention must be paid to synthetic peptides which show very weak reactivity when tested by an enzyme immunoassay.

Fortunately, the low background absorbance of synthetic peptides allows for the precise detection of weak reactivities. In some cases, absorbances of 50 mA versus background reading are of sufficient significance and can lead to the identification of important epitopes through successive refinement of the amino acid sequence of a peptide. The utmost technical skill is required to obtain consistent and reliable results when working in the range of absorbances below 200-300 mA. For example: Peptides 261E and 261F shown on Table 4D were reactive with only one of eight HCV sera panel members (Panel I), with absorbances of 307 and 269 mA, respectively. Yet this weak reactivity led to the eventual identification of pep3 (or 279A), toward which half of the panel is reactive, and toward which some additional reactive samples show absorbances of >2000 mA.

Based on the immunoreactivities of the peptides according to the present invention, it is believed that these peptides may also be useful in a vaccine to prevent NANBH. The peptide when coupled to a protein, or synthesized on a polymeric carrier resin (e.g., an octameric lysine resin) or when polymerized to homo or hetero dimers or higher oligomers by cysteine oxidation, or induced disulfide cross linking, or by use of homo or hetero functional multivalent cross linking reagents, can be introduced to normal subjects to stimulate production of antibodies to HCV in healthy mammals.

The advantages of using synthetic peptides are known.

Since the peptides according to the present invention are not derived biologically from the virus, there is no danger of exposing the normal subjects who are to be vaccinated to the disease causing pathogen.

The peptides can be chemically synthesized easily. This means that there is no involvement with HCV at any time during the process of making the test reagent or the vaccine. Another problem which can be minimized by the process of the present invention is the false positive results caused by the presence of antigenic material co-purified with the HCV fusion protein. Certain normal individuals have antibodies to E. coli or yeast proteins which are cross reactive with the antigenic materials from the expression system. Sera from these normal individuals may show a positive reaction in the immunoassays.

Further, with appropriate amino acid modification or substitutions, it is expected that various peptide analogues based on the prescribed amino acid sequence can be synthesized with properties giving rise to lower background readings or better binding capacity to solid phases useful for HCV antibody screening assays.

Moreover, because the peptide compositions of the present invention are synthetically prepared, the quality can be controlled and as a result, reproducibility of the test results can be assured. Also, since very small amounts of a peptide are required for each test procedure, and because the expense of preparing a peptide is relatively low, the cost of screening body fluids for antibodies to HCV, diagnosis of NANBH infection, and the preparation of a vaccine is relatively low.

The peptides prepared in accordance with the present invention can be used to detect HCV infection and diagnose NANBH by using them as the test reagent in an enzyme-linked immunoadsorbent assay (ELISA), an enzyme immunodot assay, an agglutination based assay, or other well-known immunosassay devices. The following examples serve to illustrate the present invention and are not to be used to limit the scope of the invention.

### EXAMPLE 1

### Measurement of Relative (%) Immunoreactivity for HCV synthetic peptides by an Enzyme-Linked Immunosorbent Assay

As an example to illustrate how relative (%) immunoreactivity for HCV synthetic peptides is measured, wells of 96-well plates are coated for 1 hour at 37°C, with each of the following peptides: IIH, V, VIIIE and pep11 at 5 ug/mL at 100 uL per well in 10mM NaHCO₃ buffer, pH 9.5. The peptide coated wells were then incubated with 250 uL of 3% by weight of gelatin in PBS in 37°C for 1 hour to block non-specific protein binding sites, followed by three washes with PBS containing 0.05% by volume of TWEEN 20 and then dried. The test specimens containing a panel of eight well-characterized HCV antibody positive patient sera were diluted with PBS containing 20% by volume normal goat serum, 1% by weight gelatin and 0.05% by volume TWEEN 20 at dilutions of 1:20 volume to volume, respectively. 200 uL of the diluted specimens were added to each of the wells and allowed to react for 15 minutes at 37°C.

The wells were then washed six times with 0.05% by volume TWEEN 20 in PBS in order to remove unbound antibodies. Horseradish peroxidase conjugated goat anti-human IgG was used as a second antibody tracer to bind with the HCV antibody-peptide antigen complex formed in positive wells. 100 uL of peroxidase labeled goat anti-human IgG at a dilution of 1:1800 in 1% by volume normal goat serum, 0.05% by volume TWEEN 20 in PBS was added to each well and incubated at 37°C for another 15 minutes.

The wells were washed six times with 0.05% by volume TWEEN 20 PBS to remove unbound antibody and reacted with 100uL of the substrate mixture containing 0.04% by weight orthophenylenediamine (OPD) and 0.12% by volume hydrogen peroxide in sodium citrate buffer, pH 5.0.

This substrate mixture was used to detect the peroxidase label by forming a colored product. Reactions were stopped by the addition of 100 uL of 1.0M H₂SO₄ and the A₄₉₂mm measured. Results of relative immunoreactivity for each of the peptides obtained from this study are shown in Table A using peptide II H as the reference.

**Table A**

| Peptide Code | A₄₉₂nm (Panel I, No. 1 to 8) | | | | | | | | | |
|---|---|---|---|---|---|---|---|---|---|---|
| | 1 | 2 | 3 | 4 | 5 | 6 | 7 | 8 | Total | % |
| IIH | 0.812 | 0.656 | 3.114 | 2.737 | 1.066 | 2.254 | 2.599 | 3.478 | 16.712 | 100 |
| V | 0.834 | 1.060 | 2.931 | 0.534 | 0.137 | 0.434 | 0.303 | 2.787 | 9.020 | 54 |
| VIIIE | 2.745 | 2.208 | 2.468 | 3.032 | 0.054 | 2.108 | 0.730 | 3.006 | 16.351 | 98 |
| Pep11 | 0.241 | 0.715 | 3.162 | 1.020 | 0.568 | 2.166 | 3.330 | 3.477 | 14.690 | 88 |

### EXAMPLE 2

### Comparison of HCV Immunoreactivities by a Well-characterized 8 Member HCV Serum Panel (Panel I) for % Relative Immunoreactivity with a Group of HCV Capsid Protein Related Peptides by an Enzyme Immunoassay

A 36mer HCV capsid peptide recently disclosed by Okamoto et al. (8) as the basis of an HCV EIA was synthesized for the purpose of comparison of immunoreactivity with peptides VIIIA, VIIIB and VIIIE (Table 2A). According to a procedure described in Example 1, peptides were coated at concentrations of 5, 1 and 0.2 µg/mL for immunopotency comparison. This 36mer exhibited only 47.8% of the reactivity of VIIIE (Table 2A). More importantly, when tested by our well-characterized HCV serum panel used for serological validation, only 4 out of 8 samples reacted with the 36mer, compared with 7 out of 8 with VIIIE. The C terminal end of this 36mer does not appear to contribute to the peptide's HCV immunoreactivity, since IXD is not greater in reactivity than IXC (Table 2A).

In addition, a 61mer peptide and fragments thereof consisting of a 30mer, a 40mer and a 50mer corresponding to sequences from Arima clone 1, which is homologous to the capsid region of the flavivirus yellow fever virus, were synthesized and compared in immunoreactivity with peptide VIIIE from the corresponding region of HCV (Table 2B). The 40mer and 61mer of clone 1 exhibited the most reactivity. However these were only 21.1% and 20.7%, respectively, of the immunoreactivity of peptide VIIIE.

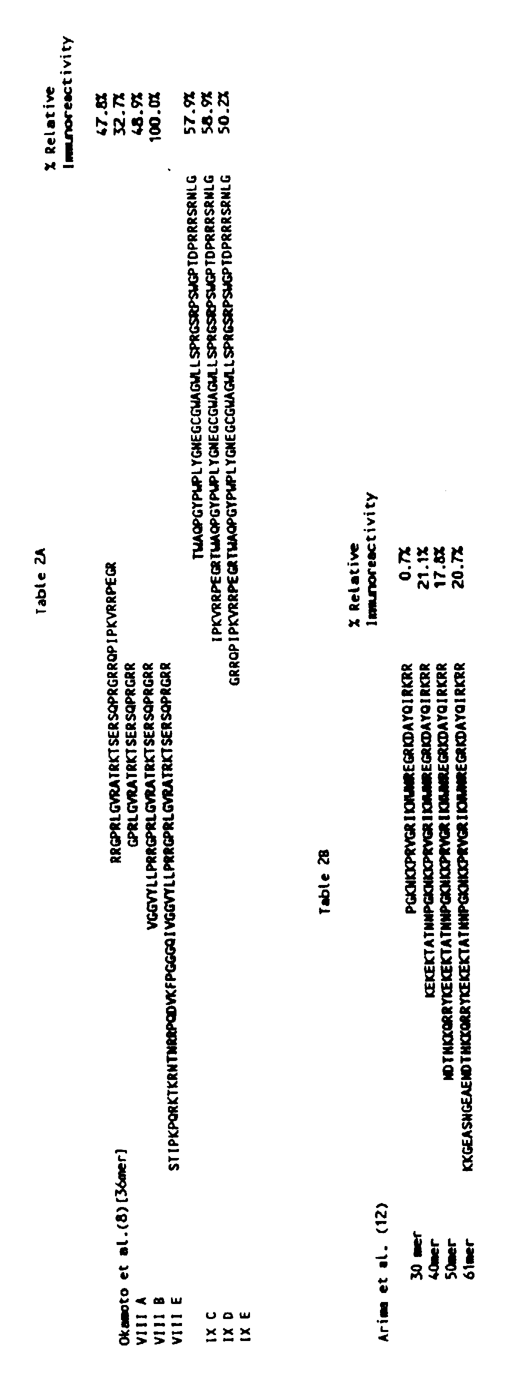

### EXAMPLE 3

### Detection of Antibodies to HCV By an Agglutination Based Assay

The presently claimed HCV peptides, synthesized according to the Merrifield solid phase method, can be conjugated to bovine serum albumin (BSA) by a simple crosslinking method in the presence of a low percentage of glutaraldehyde solution, or with other crosslinking reagent such as m-maleimidobenzoyl-N-hydroxysuccinimide ester (MBS).

Based on the above mentioned peptide-BSA conjugation process, conjugated peptide was absorbed onto double aldehyde fixed human O erythrocytes at pH 4.0. The peptide-conjugate coated erythrocytes were then treated with NaBH₄ to prevent non-specific protein binding. The peptide-cqnjugate coated erythrocytes were then washed with PBS and incubated with 5% normal human serum-PBS solution. These processed cells were then used in an agglutination assay for the detection of HCV antibodies in both serum and plasma specimens. The specimens were diluted 1:10 in a sample diluent buffer and an equal volume of the indicator cells was mixed with the diluted specimens. The agglutination pattern was settled within one hour; and the assay results were read by eye. Serial bleedings from three well-characterized HCV seroconversion panels were tested for antibodies to HCV in the above-described HCV passive hemagglutination assay (PHA) employing Peptide VIIIE-BSA conjugate and Peptide IIH-BSA conjugate as the solid phase. The results were compared with the A492 a S/C of the peptide based HCV EIA (Format C, a combination of peptides IIH, V and VIIIE) and C100 based HCV EIA (Table 3).

In brief, the PHA assay detected HCV antibodies in all three panels as early as there was an increase in A492 in the peptide based EIA (Format C). rC100 based EIA lagged behind the HCV PHA results by 4-8 weeks.

**Table 3**

| Dectection of HCV Specific Antibodies from Seroconversion Panels by Various HCV Antibody Assays | | | | | |
|---|---|---|---|---|---|
| Series | Days | ALT | Format C HCV EIA S/C Ratio | C100 Based HCV EIA | HVC PHA Visual Score |
| A* (Serologicals Panel B) | 0 | 4b | 0.108 | 0.03 | - |
| | 7 | 32 | 0.045 | 0.04 | - |
| | 14 | 32 | 0.025 | 0.06 | ++ |
| | 21 | 180 | 1.037 | 0.04 | ++ |
| | 50 | 401 | 7.193 | 0.19 | ++ |
| | 92 | - | 10.185 | 6.57 | ++ |
| | 105 | - | 9.770 | 6.57 | ++ |
| B* (Serologicals Panel A) | 0 | 39 | 0 | 0 | - |
| | 10 | 274 | 0.058 | 0 | - |
| | 14 | 346 | 0.128 | 0 | - |
| | 30 | 1175 | 7.835 | 6.5 | ++ |
| | 51 | 430 | 7.811 | 6.5 | ++ |
| C* (Serologicals Panel C) | 0 | 63 | 0.115 | 0.04 | - |
| | 2 | 81 | 1.607 | 0.04 | ++ |
| | 9 | 183 | 2.506 | 0.02 | ++++ |
| | 29 | 563 | 9.827 | 6.57 | ++++ |
| | 57 | 436 | 10.630 | 6.57 | ++++ |

| | | | | | |
|---|---|---|---|---|---|
| * Case presented is a plasma donor from a commercial source. Day 0 designates first sample in the series and does not correspond to date of exposure. | | | | | |

### Example 4

### Detection of Antibodies to HCV by an Agglutination Assay Utilizing as the Solid Phase Immunosorbent Latex Particles Coated with HCV Peptide

Using the peptide-BSA conjugation process mentioned in the previous example, conjugated peptide VIIIE-BSA, was absorbed to latex particles (0.4µ size) at pH 9.5. The peptide-conjugate coated latex particles were then treated with BSA to prevent nonspecific protein binding. These coated latex particles were then used in an agglutination assay for the detection of HCV antibodies. The specimens were mixed in a ratio of 1:1 with the latex solution (0.5%). The agglutination pattern was complete in a period of 15 min. Assay results were read by eye and by microscopic examination. The results of serial dilution samples from a well characterized anti-HCV positive plasma sample are summarized in Table 4. A coating concentration of 0.3 mg/mL was found to give optimal sensitivity for antibody detection. As a control for specificity, pooled plasma specimens from normal donors were tested in the peptide VIII-BSA conjugate latex assay and were found clearly negative.

**Table 4**

| Rapid Detection of HCV Antibodies using VIIIE-BSA Sensitized Latex Particles and Scoring for Visual Agglutination Pattern | | | | |
|---|---|---|---|---|
| | Degree of Agglutination | | | |
| HCV Positive Control Dilution | VIIIE-BSA 2.4 mg/mL | Latex Particle 1.2 mg/mL | Coating 0.6 mg/mL | Concentration 0.3 mg/mL |
| 1:1 | 4+ | 4+ | 4+ | 4+ |
| 1:2 | 4+ | 4+ | 4+ | 4+ |
| 1:5 | 4+ | 4+ | 4+ | 4+ |
| 1:10 | 4+ | 4+ | 4+ | 4+ |
| 1:20 | 3+ | 4+ | 4+ | 4+ |
| 1:40 | 2+ | 3+ | 4+ | 4+ |
| 1:80 | +/- | - | + | 3+ |
| 1:160 | - | - | - | + |
| 1:320 | - | - | - | +/- |
| 1:640 | - | - | - | +/- |
| NP 1:1 | - | - | - | - |
| NP: Pooled Normal Plasma | | | | |

### EXAMPLE 5

### SYNTHESIS OF OCTAMERIC HCV PEPTIDE ANTIGENS AS KEY COMPONENTS OF IMMUNOGENS/VACCINES

The use of a limited sequential propagation of a trifunctional amino acid (or similar analogues) to form a core that serves as a low molecular weight matrix is the basic underlying principle for the formation of a radially branching multimeric peptide antigen system. The trifunctional amino acid, Boc-Lys(Boc), or di-(Boc)-Lys is most suitable since both N^{α}- and N^{ε}- amino acid groups are available as reactive ends. Thus, sequential propagation of di-(Boc)-Lys will generate 2ⁿ reactive ends. For example, the first level coupling of di-(Boc)-Lys will produce two reactive amino ends as a bivalent peptide antigen. Sequential generations of a second, third, and fourth step with di-(Boc)-Lys will therefore generate tetravalent, octavalent, and hexadecavalent peptide antigens respectively. As an example, an octameric HCV peptide immunogen with a structure of [Gln-Gly-Trp-Gly-Pro-Ile-Ser-Tyr-Ala-Asn-Gly-Ser-Gly-Pro-Asp-Gln-Arg-Pro-Tyr-Cys-Trp-His-Tyr-Pro-Pro-Lys-Pro-Cys-Gly-Ile-Val-Pro-Ala-Lys-Ser-Val-Cys-Gly-Pro-Val-Tyr-Cys]₈-Lys₄-Lys₂-Lys was synthesized as a prototype immunogen used in our immunization of guinea pigs. This octameric antigen contains a small heptalysyl core (<20%) and the bulk (>80%) is formed by a high density of uniform peptide-antigen layered around the core matrix. This design differs from the conventional peptide-carrier conjugate which contains a large protein carrier such as PPD or KLH and a low density of peptide antigens randomly distributed on the protein carrier surface in an unidentified form.

For the synthesis of octameric HCV peptide immunogen, a combination of Boc-amino acid resin-bound benzhydrylamide and tBoc-chemistry was used. An octameric heptalysyl core resin was prepared by coupling di-t-Boc Lys onto an extra low loading 0.14 mmole/g MBHA (4-methyl benzhydrylamine) resin on a Biosearch 9500 instrument. During each of the two coupling cycles, di-(Boc)-Lys was used for single coupling followed by two capping reactions (with 0.3 M acetylimidazole in DMF dimethylformamide).

After two additional di-(Boc)-Lys couplings onto the first di-(NH₂) Lys-resin, the substitution level of synthetic octameric resin was determined by ninhydrin test and found to have an appropriate level of -NH₂ groups, as calculated based on a theoretical coupling yield, and was used thereafter for the synthesis of octameric peptide antigens each with a predefined amino acid sequence according to the standard t-Boc chemistry.

Acid-labile tert-butyloxycarbonyl (t-Boc) was used for the protection of N-α amino acid. The following functional side-chain protecting groups were used: O-benzyl for Thr, Ser, Glu and Tyr; N^{δ}-tosyl for Arg; BOM(i.e. Boc-N^{im}-Benzyloxymethyl-) for His; N^{ε}-dichlorobenzyloxycarbonyl for Lys; S-4-methylbenzyl- for Cys; O-cyclohexyl for Asp and CHO for Trp. Successive amino acids were added as dictated by the sequence. The resultant octameric peptidyl resin was cleaved by anhydrous HF [O°C for 1 hr in the presence of 10% (v/v) anisole]. The released octomeric antigen was extracted by acetic acid, after two cycles of ether washings of the cleaved peptidyl resin, and lyophilized to dryness so as to be ready for use as an immunogen. A computer-generated picture of such an octameric immunogen is shown in Fig. 1.

### EXAMPLE 6

### Detection of Antibodies to HCV by Peptide Based HCV EIA Using Formats 1 to 6

The following five groups of serum specimens:
(a) Plasmapheresis donors with elevated (>100 i.u./L) alanine aminotransferase (ALT) enzyme activity (n=30);
(b) Blood donors with elevated (>45 i.u./L) ALT enzyme activity (n=15);
(c) Chronic NANBH patients (n=30);
(d) Other viral infections (n=11);
(e) Autoimmune disease patients (n=9);
were analyzed on representative HCV peptide based EIA kits according to the present invention, with the plates coated at 100 uL per well either with:
(i) Format 1: peptides VIII E, II H and pepll at 0.5, 3 and 1 µg/mL each;
(ii) Format 2: peptides VIII E and pepll at 0.5 and 1 µg/mL each;
(iii) Format 3: peptides VIII E, pepll and peps at 0.5, 1 and 10 µg/mL each;
(iv) Format 4: peptides VIII E and pep8 at 0.5 and 10 µg/mL each;
(v) Format 5: peptides VIII E, pepll and pep12. at 0.5, 1 and 2 µg/mL each;
(vi) or Format 6: peptides VIII E and pep12 at 0.5 and 2 µg/mL each.

These kits represent core, NS-4 and NS-5 (Format 1), core and NS-5 (Formats 2, 5 and 6), core, NS-5 and env (Format 3) and core and env (Format 4).

The results of testing these 95 well characterized samples on Formats 1 through 6 are presented in Table 5. The results indicate that (30/30) of the samples in group (a) were reactive by Formats 1, 2 and 3; 90% (27/30) reactive by Format 4 and 97% (29/30) reactive by Formats 5 and 6. All samples in groups (b) and (c) were positive on all 6 formats. Groups (a), (b) and (c) were shown to be reactive by Format C, a mixture of peptides II H, V and VIII E.

Three samples in group (d) were reactive by Formats 1 to 4. In contrast, these samples were indicated as negative by Format C. Serum samples "86" and "124" apparently responded to the presence of pepll, and serum sample "VZV2500" was indicated as positive by the presence of pep8 in Formats 4 and 5.

All serum samples in group (c) were negative on all formats, including Format C.

**Table 5**

| Antibody to HCV Detected By Peptide Based EIA Kits (Absorbance 492nm) | | | | | | | |
|---|---|---|---|---|---|---|---|
| Sample ID | | Format 1 | Format 2 | Format 3 | Format 4 | Format 5 | Format 6 |
| NRC | | 0.065 | 0.075 | 0.056 | 0.061 | 0.060 | 0.019 |
| WRC | | 0.650 | 0.454 | 0.953 | 0.967 | 0.403 | 0.340 |
| SRC | | 2.183 | 1.791 | 2.580 | 2.635 | 1.589 | 1.331 |

| a. Plasmapheresis, ALT > 100 i.u.L | | | | | | | |
|---|---|---|---|---|---|---|---|
| 1 | -13 | 3.166 | 3.419 | 3.255 | 3.371 | 3.291 | 3.255 |
| | -27 | 1.555 | 1.548 | 1.980 | 2.904 | 1.152 | 0.881 |
| | -31 | 3.479 | 3.144 | 3.220 | 2.332 | 3.319 | 2.665 |
| | -32 | 3.001 | 3.035 | 3.112 | 2.691 | 3.076 | 2.986 |
| | -39 | 3.063 | 3.041 | 3.361 | 2.886 | 3.190 | 3.038 |
| | -42 | 3.198 | 3.201 | 3.050 | 3.227 | 3.230 | 3.118 |
| | -47 | 3.479 | 3.110 | 3.251 | 3.201 | 3.229 | 3.068 |
| | -48 | 3.142 | 2.795 | 3.116 | 2.934 | 3.076 | 2.725 |
| | -49 | 3.417 | 3.291 | 3.525 | 3.451 | 3.195 | 3.592 |
| | -52 | 3.263 | 3.329 | 3.202 | 0.120 | 3.262 | 3.453 |
| | -53 | 3.225 | 3.145 | 3.096 | 0.062 | 3.358 | 3.097 |
| | -54 | 3.271 | 3.018 | 3.267 | 0.153 | 3.073 | 3.211 |
| 2 | -4 | 1.012 | 0.881 | 1.542 | 1.767 | 0.807 | 0.745 |
| | -6 | 3.229 | 2.964 | 3.169 | 3.052 | 3.076 | 2.897 |
| | -9 | 2.691 | 2.416 | 2.766 | 2.967 | 2.119 | 1.844 |
| | -26 | 3.222 | 3.055 | 3.095 | 3.167 | 3.195 | 2.951 |
| | -32 | 3.226 | 3.372 | 3.368 | 3.194 | 3.496 | 3.417 |
| | -33 | 3.151 | 2.918 | 3.147 | 3.027 | 3.108 | 3.129 |
| | -34 | 3.059 | 3.021 | 3.143 | 3.167 | 3.145 | 3.320 |
| | -38 | 3.241 | 3.116 | 2.967 | 3.055 | 3.213 | 3.137 |
| | -41 | 2.964 | 2.593 | 2.841 | 2.964 | 2.469 | 2.252 |
| | -43 | 3.146 | 2.092 | 2.541 | 2.627 | 1.999 | 1.920 |
| | -46 | 2.927 | 2.818 | 2.998 | 2.983 | 2.556 | 2.415 |
| | -58 | 3.285 | 3.444 | 3.218 | 3.191 | 3.355 | 3.095 |
| | -60 | 3.094 | 2.975 | 3.113 | 3.167 | 2.683 | 2.640 |
| | -61 | 2.784 | 2.345 | 2.501 | 2.751 | 2.007 | 2.212 |
| | -62 | 3.320 | 3.076 | 3.095 | 3.076 | 3.003 | 2.787 |
| | -77 | 0.815 | 0.682 | 1.096 | 0.418 | 0.164 | 0.152 |
| | -82 | 3.020 | 2.982 | 1.826 | 3.001 | 3.032 | 2.820 |
| | -83 | 3.076 | 2.914 | 3.049 | 2.996 | 2.928 | 2.808 |

| b. Elevated ALT blood donors (ALT > i.u./L) | | | | | | | |
|---|---|---|---|---|---|---|---|
| ALT | -1 | 3.017 | 3.035 | 3.116 | 3.165 | 3.167 | 2.920 |
| | -2 | 3.256 | 3.166 | 3.165 | 2.974 | 3.292 | 3.091 |
| | -3 | 3.153 | 3.328 | 3.291 | 3.105 | 3.203 | 3.230 |
| | -4 | 2.969 | 2.894 | 3.096 | 3.144 | 2.880 | 2.866 |
| | -5 | 3.073 | 2.956 | 2.968 | 2.952 | 3.376 | 2.985 |
| | -7 | 3.218 | 3.020 | 3.157 | 2.980 | 2.951 | 3.060 |
| | -8 | 3.074 | 2.930 | 3.094 | 3.197 | 3.121 | 3.012 |
| | -10 | 3.479 | 3.228 | 3.226 | 3.109 | 3.432 | 3.952 |
| | -11 | 3.398 | 3.283 | 3.140 | 3.035 | 3.285 | 3.222 |
| | -53 | 3.330 | 3.029 | 3.253 | 3.290 | 2.974 | 3.070 |
| | -56 | 3.151 | 3.086 | 3.176 | 3.202 | 3.107 | 3.085 |
| | -69 | 3.021 | 3.170 | 3.167 | 3.318 | 3.019 | 2.831 |
| | -70 | 3.074 | 3.035 | 2.951 | 3.073 | 3.054 | 3.184 |
| | -71 | 2.985 | 2.901 | 3.080 | 3.039 | 2.902 | 2.900 |
| | -82 | 3.230 | 3.120 | 3.085 | 2.977 | 3.298 | 3.096 |

| c. Chronic NANBH | | | | | | | |
|---|---|---|---|---|---|---|---|
| N | -2 | 3.320 | 3.052 | 2.981 | 3.283 | 3.032 | 2.999 |
| | -3 | 3.285 | 3.036 | 3.095 | 3.167 | 3.077 | 3.094 |
| | -4 | 3.117 | 3.469 | 3.590 | 3.291 | 2.259 | 3.141 |
| | -7 | 3.027 | 3.008 | 3.061 | 3.065 | 2.962 | 2.806 |
| | -8 | 3.285 | 3.146 | 3.117 | 3.194 | 3.122 | 3.195 |
| | -9 | 2.886 | 3.001 | 3.072 | 2.985 | 2.848 | 2.859 |
| | -10 | 2.606 | 2.268 | 2.027 | 0.423 | 2.338 | 1.104 |
| | -14 | 3.054 | 2.808 | 2.856 | 2.995 | 2.341 | 2.041 |
| | -23 | 3.228 | 3.050 | 3.067 | 3.225 | 3.152 | 3.109 |
| | -25 | 3.891 | 2.462 | 3.190 | 3.165 | 1.982 | 2.091 |
| | -27 | 3.194 | 2.926 | 3.165 | 3.029 | 3.143 | 3.168 |
| | -28 | 3.027 | 3.106 | 3.259 | 3.175 | 3.176 | 3.202 |
| | -34 | 3.057 | 3.037 | 3.035 | 3.144 | 2.907 | 2.892 |
| | -36 | 3.304 | 3.213 | 3.000 | 3.033 | 3.075 | 3.115 |
| | -41 | 3.217 | 3.283 | 3.039 | 3.248 | 3.290 | 3.249 |
| | -42 | 2.997 | 2.858 | 3.196 | 3.094 | 3.097 | 2.805 |
| | -44 | 3.391 | 3.477 | 3.350 | 3.254 | 3.353 | 3.387 |
| | -45 | 3.318 | 3.096 | 2.964 | 3.250 | 3.319 | 3.036 |
| | -49 | 3.292 | 3.371 | 3.416 | 3.255 | 3.292 | 3.370 |
| | -54 | 3.329 | 3.294 | 3.105 | 3.105 | 3.177 | 3.203 |
| | -57 | 3.197 | 3.169 | 3.221 | 3.141 | 3.120 | 3.018 |
| | -60 | 3.115 | 3.035 | 3.090 | 3.072 | 3.096 | 2.873 |
| | -65 | 2.020 | 1.816 | 1.898 | 2.376 | 1.133 | 1.284 |
| | -67 | 2.265 | 1.776 | 2.356 | 2.396 | 1.319 | 0.911 |
| | -68 | 3.178 | 3.177 | 3.200 | 3.176 | 3.530 | 3.087 |
| | -69 | 3.222 | 3.167 | 3.165 | 3.283 | 3.399 | 3.097 |
| | -77 | 1.438 | 1.346 | 2.548 | 2.397 | 1.055 | 1.071 |
| | -78 | 2.457 | 2.038 | 2.251 | 2.300 | 1.642 | 1.494 |
| | -79 | 3.225 | 3.197 | 3.076 | 3.142 | 3.224 | 3.169 |
| | -80 | 3.138 | 3.074 | 3.135 | 3.054 | 3.137 | 2.896 |

| d. Other Viral Infections | | | | | | | |
|---|---|---|---|---|---|---|---|
| HAV | -86 | 0.558 | 0.316 | 0.607 | 0.054 | 0.037 | 0.014 |
| | -88 | 0.018 | 0.021 | 0.062 | 0.054 | 0.014 | 0.018 |
| | -92 | 0.045 | 0.061 | 0.058 | 0.050 | 0.043 | 0.007 |
| | -120 | 0.057 | 0.076 | 0.051 | 0.032 | 0.051 | 0.026 |
| | -121 | 0.052 | 0.138 | 0.094 | 0.065 | 0.072 | 0.026 |
| | -124 | 0.816 | 1.178 | 0.622 | 0.062 | 1.082 | 0.017 |
| | -125 | 0.014 | 0.016 | 0.050 | 0.031 | 0.012 | 0.010 |
| | -126 | 0.105 | 0.134 | 0.109 | 0.081 | 0.117 | 0.068 |
| EBV | -2331 | 0.021 | 0.021 | 0.023 | 0.020 | 0.012 | 0.012 |
| VZV | -M002 | 0.035 | 0.030 | 0.154 | 0.108 | 0.025 | 0.012 |
| VZV | -2500 | 0.090 | 0.138 | 0.976 | 0.923 | 0.084 | 0.032 |

| e. Autoimmune | | | | | | | |
|---|---|---|---|---|---|---|---|
| | -209 | 0.102 | 0.079 | 0.117 | 0.097 | 0.066 | 0.028 |
| | -210 | 0.002 | 0.003 | 0.018 | 0.011 | 0.002 | 0.005 |
| | -211 | 0.016 | 0.019 | 0.134 | 0.168 | 0.022 | 0.016 |
| | -212 | 0.016 | 0.020 | 0.075 | 0.080 | 0.019 | 0.006 |
| | -213 | 0.008 | 0.009 | 0.055 | 0.076 | 0.005 | 0.002 |
| | -215 | 0.118 | 0.095 | 0.226 | 0.282 | 0.093 | 0.060 |
| | -216 | 0.039 | 0.037 | 0.100 | 0.105 | 0.042 | 0.022 |
| | -217 | 0.019 | 0.021 | 0.068 | 0.056 | 0.023 | 0.012 |
| | -218 | 0.032 | 0.022 | 0.110 | 0.086 | 0.059 | 0.031 |

### EXAMPLE 7

### Comparison of Test Results Using the Six Peptide Based HCV EIA Formats (1-6) on Random Blood Donors

Random blood donor samples (n=100) were tested by Formats 1 to 6. All 100 samples were negative on Formats 2, 5 and 6. Sample 14 had an absorbance of 0.680 on Format 1, and sample 34 had an absorbance of 0.601 and 0.551 on Formats 3 and 4, respectively. For the calculation of mean absorbance and standard deviation, absorbance values >0.500 were omitted from analysis. Table 6 lists the mean absorbance and standard deviation of the 100 samples on Formats 1-6.

**Table 6**

| Mean Absorbance (A492nm) ± SD of 100 Random Blood Donors | | | | | | |
|---|---|---|---|---|---|---|
| | Format 1 | Format 2 | Format 3 | Format 4 | Format 5 | Format 6 |
| Mean | 0.040 | 0.035 | 0.068 | 0.061 | 0.030 | 0.017 |
| S.D. | 0.036 | 0.029 | 0.046 | 0.046 | 0.039 | 0.032 |

### EXAMPLE 8

### Comparison of Immunoreactivity for NS-5 Protein Derived Synthetic Peptides

Wells of 96-well plates were coated for 1 hour at 37°C with each of the 23 peptides (designated as 259A-259E, 260A-260C, 309A-309C, 310A-310C, 311A-311C, 312A-312C and 314A-314C) synthesized with sequences derived from the NS-5 region, at 5 µg/mL at 100 µL per well in 10 mM NaHCO₃ buffer, pH 9.5. The immunoreactivity of each peptide was measured by an 8 member HCV serum panel (Panel I). The peptide with the greatest immunoreactivity was pepll, designated 309C in Table 7. When the immunoreactivity of pep11 was used as a standard to calculate the relative immunopotency for the other NS-5 peptides, the peptides in series 309-314 were seen to be equal to or more reactive than pep4 and pep5. The extension of pep5 to include an additional 10 residues (259E, i.e. pepl2) increased the relative immunopotency from 47.6% to 70.1%.

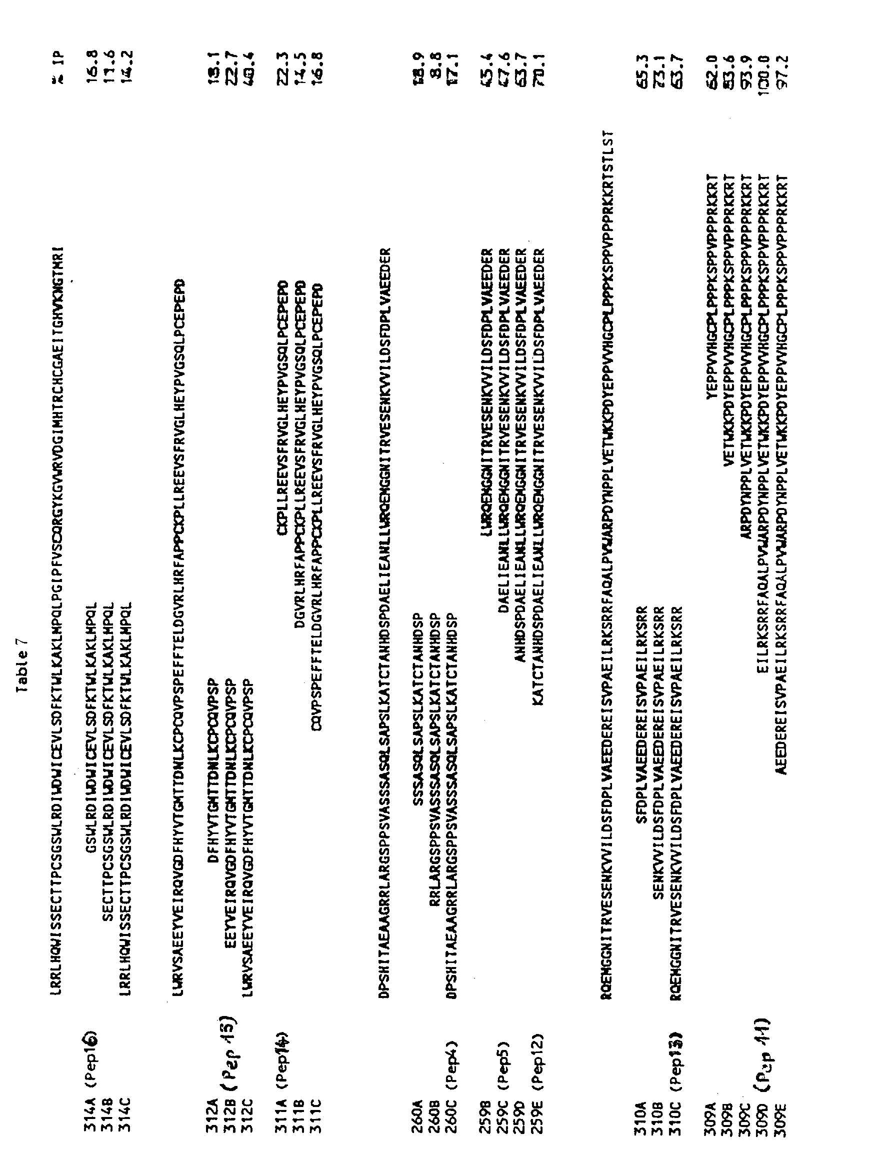

### SEQUENCE LISTING

SEQ ID No.: **241A**
   Sequence Type: amino acid (AA)
   Sequence Length: 37 AA 
Gln-Gly-Trp-Gly-Pro-Ile-Ser-Tyr-Ala-Asn-Gly-Ser-Gly-Pro-Asp-
                5                   10                  15
Gln-Arg-Pro-Tyr-Cys-Trp-His-Tyr-Pro-Pro-Lys-Pro-Cys-Gly-Ile-
                20                  25                  30
Val-Pro-Ala-Lys-Ser-Val-Cys
                35
SEQ ID No.: **241B**
   Sequence Type: AA
   Sequence Length: 45 AA 
Cys-Arg-Pro-Leu-Thr-Asp-Phe-Asp-Gln-Gly-Trp-Gly-Pro-Ile-Ser-
                5                   10                  15
Tyr-Ala-Asn-Gly-Ser-Gly-Pro-Asp-Gln-Arg-Pro-Tyr-Cys-Trp-His-
                20                  25                  30
Tyr-Pro-Pro-Lys-Pro-Cys-Gly-Ile-Val-Pro-Ala-Lys-Ser-Val-Cys-
                35                  40                  45
SEQ ID No.: **241C**
   Sequence Type: AA
   Sequence Length: 52 AA 
Cys-Pro-Glu-Arg-Leu-Ala-Ser-Cys-Arg-Pro-Leu-Thr-Asp-Phe-Asp-
5 10 15
Gln-Gly-Trp-Gly-Pro-Ile-Ser-Tyr-Ala-Asn-Gly-Ser-Gly-Pro-Asp-
20 25 30
Gln-Arg-Pro-Tyr-Cys-Trp-His-Tyr-Pro-Pro-Lys-Pro-Cys-Gly-Ile-
35 40 45
Val-Pro-Ala-Lys-Ser-Val-Cys
50
SEQ ID No.: **231A**
   Sequence Type: AA
   Sequence Length: 26 AA 
Arg-Pro-Tyr-Cys-Trp-His-Tyr-Pro-Pro-Lys-Pro-Cys-Gly-Ile-Val-
                5                   10                  15
Pro-Ala-Lys-Ser-Val-Cys-Gly-Pro-Val-Tyr-Cys
                20                  25
SEQ ID No.: **231B**
   Sequence Type: AA
   Sequence Length: 34 AA 
Ala-Asn-Gly-Ser-Gly-Pro-Asp-Gln-Arg-Pro-Tyr-Cys-Trp-His-Tyr-
                    5                   10                  15
Pro-Pro-Lys-Pro-Cys-Gly-Ile-Val-Pro-Ala-Lys-Ser-Val-Cys-Gly-
                    20                  25                  30
Pro-Val-Tyr-Cys
SEQ ID No.: **231C (Pep 1)**
   Sequence Type: AA
   Sequence Length: 42 AA 
Gln-Gly-Trp-Gly-Pro-Ile-Ser-Tyr-Ala-Asn-Gly-Ser-Gly-Pro-Asp-
                5                   10                  15
Gln-Arg-Pro-Tyr-Cys-Trp-His-Tyr-Pro-Pro-Lys-Pro-Cys-Gly-Ile-
                20                  25                  30
Val-Pro-Ala-Lys-Ser-Val-Cys-Gly-Pro-Val-Tyr-Cys
                35                  40
SEQ ID No.: **231D**
   Sequence Type: AA
   Sequence Length: 50 AA 
Cys-Arg-Pro-Leu-Thr-Asp-Phe-Asp-Gln-Gly-Trp-Gly-Pro-Ile-Ser-
                5                   10                  15
Tyr-Ala-Asn-Gly-Ser-Gly-Pro-Asp-Gln-Arg-Pro-Tyr-Cys-Trp-His-
                20                  25                  30
Tyr-Pro-Pro-Lys-Pro-Cys-Gly-Ile-Val-Pro-Ala-Lys-Ser-Val-Cys-
                35                  40                  45
Gly-Pro-Val-Tyr-Cys
                50
SEQ ID No.: **231E**
   Sequence Type: AA
   Sequence Length: 57 AA 
Cys-Pro-Glu-Arg-Leu-Ala-Ser-Cys-Arg-Pro-Leu-Thr-Asp-Phe-Asp-
                5                   10                  15
Gln-Gly-Trp-Gly-Pro-Ile-Ser-Tyr-Ala-Asn-Gly-Ser-Gly-Pro-Asp-
                20                  25                  30
Gln-Arg-Pro-Tyr-Cys-Trp-His-Tyr-Pro-Pro-Lys-Pro-Cys-Gly-Ile-
                35                  40                  45
Val-Pro-Ala-Lys-Ser-Val-Cys-Gly-Pro-Val-Tyr-Cys
                50                  55
SEQ ID No.: **232A (Pep 2)**
   Sequence Type: AA
   Sequence Length: 26 AA 
Pro-Pro-Leu-Gly-Asn-Trp-Phe-Gly-Cys-Thr-Trp-Met-Asn-Ser-Thr-
                5                   10                  15
Gly-Phe-Thr-Lys-Val-Cys-Gly-Ala-Pro-Pro-Cys
                20                  25
SEQ ID No.: **232B**
   Sequence Type: AA
   Sequence Length: 34 AA 
Val-Phe-Val-Leu-Asn-Asn-Thr-Arg-Pro-Pro-Leu-Gly-Asn-Trp-Phe-
                    5                        10                       15
Gly-Cys-Thr-Trp-Met-Asn-Ser-Thr-Gly-Phe-Thr-Lys-Val-Cys-Gly-
                    20                       25                       30
Ala-Pro-Pro-Cys
SEQ ID No.: **232C**
   Sequence Type: AA
   Sequence Length: 42 AA 
Ser-Trp-Gly-Glu-Asn-Asp-Thr-Asp-Val-Phe-Val-Leu-Asn-Asn-Thr-
                5                   10                  15
Arg-Pro-Pro-Leu-Gly-Asn-Trp-Phe-Gly-Cys-Thr-Trp-Met-Asn-Ser-
                20                  25                  30
Thr-Gly-Phe-Thr-Lys-Val-Cys-Gly-Ala-Pro-Pro-Cys
                35                  40
SEQ ID No.: **232D**
   Sequence Type: AA
   Sequence Length: 50 AA 
Asp-Arg-Ser-Gly-Ala-Pro-Thr-Tyr-Ser-Trp-Gly-Glu-Asn-Asp-Thr-
                5                   10                  15
Asp-Val-Phe-Val-Leu-Asn-Asn-Thr-Arg-Pro-Pro-Leu-Gly-Asn-Trp-
                20                  25                  30
Phe-Gly-Cys-Thr-Trp-Met-Asn-Ser-Thr-Gly-Phe-Thr-Lys-Val-Cys-
                35                  40                  45
Gly-Ala-Pro-Pro-Cys
                50
SEQ ID No.: **233C**
   Sequence Type: AA
   Sequence Length: 42 AA 
Leu-His-Cys-Pro-Thr-Asp-Cys-Phe-Arg-Lys-His-Pro-Asp-Ala-Thr-
5 50 15
Tyr-Ser-Arg-Cys-Gly-Ser-Gly-Pro-Trp-Ile-Thr-Pro-Arg-Cys-Leu-
20 25 30
Val-Asp-Tyr-Pro-Tyr-Arg-Leu-Trp-His-Trp-Pro-Cys
35 40
SEQ ID No.: **234A**
   Sequence Type: AA
   Sequence Length: 23 AA 
Glu-Ala-Ala-Cys-Asn-Trp-Thr-Arg-Gly-Glu-Arg-Cys-Asp-Leu-Glu-
                5                   10                  15
Asp-Arg-Asp-Arg-Ser-Glu-Leu-Ser
                20
SEQ ID No.: **234B**
   Sequence Type: AA
   Sequence Length: 31 AA 
Val-Gly-Gly-Val-Glu-His-Arg-Leu-Glu-Ala-Ala-Cys-Asn-Trp-Thr-
5 10 15
Arg-Gly-Glu-Arg-Cys-Asp-Leu-Glu-Asp-Arg-Asp-Arg-Ser-Glu-Leu-
20 25 30
Ser
SEQ ID No.: **234C**
   Sequence Type: AA
   Sequence Length: 39 AA 
Thr-Ile-Phe-Lys-Ile-Arg-Met-Tyr-Val-Gly-Gly-Val-Glu-His-Arg-
5 10 15
Leu-Glu-Ala-Ala-Cys-Asn-Trp-Thr-Arg-Gly-Glu-Arg-Cys-Asp-Leu-
20 25 30
Glu-Asp-Arg-Asp-Arg-Ser-Glu-Leu-Ser
35
SEQ ID No.: **272A**
   Sequence Type: AA
   Sequence Length: 41 AA 
Pro-Val-Val-Pro-Gln-Ser-Phe-Gln-Val-Ala-His-Leu-His-Ala-Pro-
                5                   10                  15
Thr-Gly-Ser-Gly-Lys-Ser-Thr-Lys-Val-Pro-Ala-Ala-Tyr-Ala-Ala-
                20                  25                  30
Gln-Gly-Tyr-Lys-Val-Leu-Val-Leu-Asn-Pro-Ser
                35                  40
SEQ ID No.: **272B**
   Sequence Type: AA
   Sequence Length: 55 AA 
Thr-Thr-Met-Arg-Ser-Pro-Val-Phe-Thr-Asp-Asn-Ser-Ser-Pro-Pro-
5 10 15
Val-Val-Pro-Gln-Ser-Phe-Gln-Val-Ala-His-Leu-His-Ala-Pro-Thr-
20 25 30
Gly-Ser-Gly-Lys-Ser-Thr-Lys-Val-Pro-Ala-Ala-Tyr-Ala-Ala-Gln-
35 40 45
Gly-Tyr-Lys-Val-Leu-Val-Leu-Asn-Pro-Ser
50 55
SEQ ID No.: **272C**
   Sequence Type: AA
   Sequence Length: 66 AA 
Ala-Val-Asp-Phe-Ile-Pro-Val-Glu-Asn-Leu-Glu-Thr-Thr-Met-Arg-
5 10 15
Ser-Pro-Val-Phe-Thr-Asp-Asn-Ser-Ser-Pro-Pro-Val-Val-Pro-Gln-
20 25 30
Ser-Phe-Gln-Val-Ala-His-Leu-His-Ala-Pro-Thr-Gly-Ser-Gly-Lys-
35 40 45
Ser-Thr-Lys-Val-Pro-Ala-Ala-Tyr-Ala-Ala-Gln-Gly-Tyr-Lys-Val-
50 55 60
Leu-Val-Leu-Asn-Pro-Ser
65
SEQ ID No.: **278A**
   Sequence Type: AA
   Sequence Length: 29 AA 
Pro-Val-Val-Pro-Gln-Ser-Phe-Gln-Val-Ala-His-Leu-His-Ala-Pro-
                5                   10                  15
Thr-Gly-Ser-Gly-Lys-Ser-Thr-Lys-Val-Pro-Ala-Ala-Tyr-Ala
                20                  25
SEQ ID No.: **278B**
   Sequence Type: AA
   Sequence Length: 36 AA 
Phe-Thr-Asp-Asn-Ser-Ser-Pro-Pro-Val-Val-Pro-Gln-Ser-Phe-Gln-
5 10 15
Val-Ala-His-Leu-His-Ala-Pro-Thr-Gly-Ser-Gly-Lys-Ser-Thr-Lys-
20 25 30
Val-Pro-Ala-Ala-Tyr-Ala
35
SEQ ID No.: **278C**
   Sequence Type: AA
   Sequence Length: 48 AA 
Val-Glu-Asn-Leu-Glu-Thr-Thr-Met-Arg-Ser-Pro-Val-Phe-Thr-Asp-
                5                   10                  15
Asn-Ser-Ser-Pro-Pro-Val-Val-Pro-Gln-Ser-Phe-Gln-Val-Ala-His-
                20                  25                  30
Leu-His-Ala-Pro-Thr-Gly-Ser-Gly-Lys-Ser-Thr-Lys-Val-Pro-Ala-
                35                  40                  45
Ala-Tyr-Ala
SEQ ID No.: **278D**
   Sequence Type: AA
   Sequence Length: 54 AA 
Ala-Val-Asp-Phe-Ile-Pro-Val-Glu-Asn-Leu-Glu-Thr-Thr-Met-Arg-
5 10 15
Ser-Pro-Val-Phe-Thr-Asp-Asn-Ser-Ser-Pro-Pro-Val-Val-Pro-Gln-
20 25 30
Ser-Phe-Gln-Val-Ala-His-Leu-His-Ala-Pro-Thr-Gly-Ser-Gly-Lys-
35 40 45
Ser-Thr-Lys-Val-Pro-Ala-Ala-Tyr-Ala
50
SEQ ID No.: **275A**
   Sequence Type: AA
   Sequence Length: 38 AA 
Arg-Thr-Ile-Thr-Thr-Gly-Ser-Pro-Ile-Thr-Tyr-Ser-Thr-Tyr-Gly-
                5                   10                  15
Lys-Phe-Leu-Ala-Asp-Gly-Gly-Cys-Ser-Gly-Gly-Ala-Tyr-Asp-Ile-
                20                  25                  30
Ile-Ile-Cys-Asp-Glu-Cys-His-Ser
                35
SEQ ID No.: **275B**
   Sequence Type: AA
   Sequence Length: 71 AA 
Lys-Val-Leu-Val-Leu-Asn-Pro-Ser-Val-Ala-Ala-Thr-Leu-Gly-Phe-
                5                   10                  15
Gly-Ala-Tyr-Met-Ser-Lys-Ala-His-Gly-Ile-Asp-Pro-Asn-Ile-Arg-
                20                  25                  30
Thr-Gly-Val-Arg-Thr-Ile-Thr-Thr-Gly-Ser-Pro-Ile-Thr-Tyr-Ser-
                35                  40                  45
Thr-Tyr-Gly-Lys-Phe-Leu-Ala-Asp-Gly-Gly-Cys-Ser-Gly-Gly-Ala-
                50                  55                  60
Tyr-Asp-Ile-Ile-Ile-Cys-Asp-Glu-Cys-His-Ser
                65                  70
SEQ ID No.: **275C**
   Sequence Type: AA
   Sequence Length: 94 AA 
His-Leu-His-Ala-Pro-Thr-Gly-Ser-Gly-Lys-Ser-Thr-Lys-Val-Pro-
                5                   10                  15
Ala-Ala-Tyr-Ala-Ala-Gln-Gly-Tyr-Lys-Val-Leu-Val-Leu-Asn-Pro-
                20                  25                  30
Ser-Val-Ala-Ala-Thr-Leu-Gly-Phe-Gly-Ala-Tyr-Met-Ser-Lys-Ala-
                35                  40                  45
His-Gly-Ile-Asp-Pro-Asn-Ile-Arg-Thr-Gly-Val-Arg-Thr-Ile-Thr-
                50                  55                  60
Thr-Gly-Ser-Pro-Ile-Thr-Tyr-Ser-Thr-Tyr-Gly-Lys-Phe-Leu-Ala-
                65                  70                  75
Asp-Gly-Gly-Cys-Ser-Gly-Gly-Ala-Tyr-Asp-Ile-Ile-Ile-Cys-Asp-
                80                  85                  90
Glu-Cys-His-Ser
SEQ ID No.: **275D**
   Sequence Type: AA
   Sequence Length: 117 AA 
Thr-Met-Arg-Ser-Pro-Val-Phe-Thr-Asp-Asn-Ser-Ser-Pro-Pro-Val-
5 10 15
Val-Pro-Gln-Ser-Phe-Gln-Val-Ala-His-Leu-His-Ala-Pro-Thr-Gly-
20 25 30
Ser-Gly-Lys-Ser-Thr-Lys-Val-Pro-Ala-Ala-Tyr-Ala-Ala-Gln-Gly-
35 40 45
Tyr-Lys-Val-Leu-Val-Leu-Asn-Pro-Ser-Val-Ala-Ala-Thr-Leu-Gly-
50 55 60
Phe-Gly-Ala-Tyr-Met-Ser-Lys-Ala-His-Gly-Ile-Asp-Pro-Asn-Ile-
65 70 75
Arg-Thr-Gly-Val-Arg-Thr-Ile-Thr-Thr-Gly-Ser-Pro-Ile-Thr-Tyr-
80 85 90
Ser-Thr-Tyr-Gly-Lys-Phe-Leu-Ala-Asp-Gly-Gly-Cys-Ser-Gly-Gly-
95 100 105
Ala-Tyr-Asp-Ile-Ile-Ile-Cys-Asp-Glu-Cys-His-Ser
110 115
SEQ ID No.: **274A**
   Sequence Type: AA
   Sequence Length: 37 AA 
Thr-Val-Leu-Asp-Gln-Ala-Glu-Thr-Ala-Gly-Ala-Arg-Leu-Val-Val-
                5                   10                  15
Leu-Ala-Thr-Ala-Thr-Pro-Pro-Gly-Ser-Val-Thr-Val-Pro-His-Pro-
                20                  25                  30
Asn-Ile-Glu-Glu-Val-Ala-Leu
                35
SEQ ID No.: **274B**
   Sequence Type: AA
   Sequence Length: 64AA 
Gly-Cys-Ser-Gly-Gly-Ala-Tyr-Asp-Ile-Ile-Ile-Cys-Asp-Glu-Cys-
                5                   10                  15
His-Ser-Thr-Asp-Ala-Thr-Ser-Ile-Leu-Gly-Ile-Gly-Thr-Val-Leu-
                20                  25                  30
Asp-Gln-Ala-Glu-Thr-Ala-Gly-Ala-Arg-Leu-Val-Val-Leu-Ala-Thr-
                35                  40                  45
Ala-Thr-Pro-Pro-Gly-Ser-Val-Thr-Val-Pro-His-Pro-Asn-Ile-Glu-
                50                  55                  60
Glu-Val-Ala-Leu
SEQ ID No.: **274C**
   Sequence Type: AA
   Sequence Length: 97 AA 
Ala-His-Gly-Ile-Asp-Pro-Asn-Ile-Arg-Thr-Gly-Val-Arg-Thr-Ile-
                    5                             10                            15
Thr-Thr-Gly-Ser-Pro-Ile-Thr-Tyr-Ser-Thr-Tyr-Gly-Lys-Phe-Leu-
                    20                            25                            30
Ala-Asp-Gly-Gly-Cys-Ser-Gly-Gly-Ala-Tyr-Asp-Ile-Ile-Ile-Cys-
                    35                            40                            45
Asp-Glu-Cys-His-Ser-Thr-Asp-Ala-Thr-Ser-Ile-Leu-Gly-Ile-Gly-
                    50                            55                            60
Thr-Val-Leu-Asp-Gln-Ala-Glu-Thr-Ala-Gly-Ala-Arg-Leu-Val-Val-
                    65                            70                            75
Leu-Ala-Thr-Ala-Thr-Pro-Pro-Gly-Ser-Val-Thr-Val-Pro-His-Pro-
                    80                            85                            90
Asn-Ile-Glu-Glu-Val-Ala-Leu
                    95
SEQ ID No.: **274D**
   Sequence Type: AA
   Sequence Length: 120 AA 
Gly-Tyr-Lys-Val-Leu-Val-Leu-Asn-Pro-Ser-Val-Ala-Ala-Thr-Leu-
5 10 15
Gly-Phe-Gly-Ala-Tyr-Met-Ser-Lys-Ala-His-Gly-Ile-Asp-Pro-Asn-
20 25 30
Ile-Arg-Thr-Gly-Val-Arg-Thr-Ile-Thr-Thr-Gly-Ser-Pro-Ile-Thr-
35 40 45
Tyr-Ser-Thr-Tyr-Gly-Lys-Phe-Leu-Ala-Asp-Gly-Gly-Cys-Ser-Gly-
50 55 60
Gly-Ala-Tyr-Asp-Ile-Ile-Ile-Cys-Asp-Glu-Cys-His-Ser-Thr-Asp-
65 70 75
Ala-Thr-Ser-Ile-Leu-Gly-Ile-Gly-Thr-Val-Leu-Asp-Gln-Ala-Glu-
80 85 90
Thr-Ala-Gly-Ala-Arg-Leu-Val-Val-Leu-Ala-Thr-Ala-Thr-Pro-Pro-
95 100 105
Gly-Ser-Val-Thr-Val-Pro-His-Pro-Asn-Ile-Glu-Glu-Val-Ala-Leu
110 115 120
SEQ ID No.: **262A**
   Sequence Type: AA
   Sequence Length: 29 AA 
Tyr-Gly-Lys-Phe-Leu-Ala-Asp-Gly-Gly-Cys-Ser-Gly-Gly-Ala-Tyr-
                5                   10                  15
Asp-Ile-Ile-Ile-Cys-Asp-Glu-Cys-His-Ser-Thr-Asp-Ala-Thr
                20                  25
SEQ ID No.: **262B**
   Sequence Type: AA
   Sequence Length: 39 AA 
Thr-Thr-Gly-Ser-Pro-Ile-Thr-Tyr-Ser-Thr-Tyr-Gly-Lys-Phe-Leu-
5 10 15
Ala-Asp-Gly-Gly-Cys-Ser-Gly-Gly-Ala-Tyr-Asp-Ile-Ile-Ile-Cys-
20 25 30
Asp-Glu-Cys-His-Ser-Thr-Asp-Ala-Thr
35
SEQ ID No.: **262C**
   Sequence Type: AA
   Sequence Length: 49 AA 
Pro-Asn-Ile-Arg-Thr-Gly-Val-Arg-Thr-Ile-Thr-Thr-Gly-Ser-Pro-
                5                   10                  15
Ile-Thr-Tyr-Ser-Thr-Tyr-Gly-Lys-Phe-Leu-Ala-Asp-Gly-Gly-Cys-
                20                  25                  30
Ser-Gly-Gly-Ala-Tyr-Asp-Ile-Ile-Ile-Cys-Asp-Glu-Cys-His-Ser-
                35                  40                  45
Thr-Asp-Ala-Thr
SEQ ID No.: **262D**
   Sequence Type: AA
   Sequence Length: 59 AA 
Ala-Tyr-Met-Ser-Lys-Ala-His-Gly-Ile-Asp-Pro-Asn-Ile-Arg-Thr-
                5                   10                  15
Gly-Val-Arg-Thr-Ile-Thr-Thr-Gly-Ser-Pro-Ile-Thr-Tyr-Ser-Thr-
                20                  25                  30
Tyr-Gly-Lys-Phe-Leu-Ala-Asp-Gly-Gly-Cys-Ser-Gly-Gly-Ala-Tyr-
                35                  40                  45
Asp-Ile-Ile-Ile-Cys-Asp-Glu-Cys-His-Ser-Thr-Asp-Ala-Thr
                50                  55
SEQ ID No.: **262E**
   Sequence Type: AA
   Sequence Length: 68 AA 
Ser-Val-Ala-Ala-Thr-Leu-Gly-Phe-Gly-Ala-Tyr-Met-Ser-Lys-Ala-
                5                   10
His-Gly-Ile-Asp-Pro-Asn-Ile-Arg-Thr-Gly-Val-Arg-Thr-Ile-Thr-
                20                  25                  30
Thr-Gly-Ser-Pro-Ile-Thr-Tyr-Ser-Thr-Tyr-Gly-Lys-Phe-Leu-Ala-
                35                  40                  45
Asp-Gly-Gly-Cys-Ser-Gly-Gly-Ala-Tyr-Asp-Ile-Ile-Ile-Cys-Asp-
                50                  55                  60
Glu-Cys-His-Ser-Thr-Asp-Ala-Thr
                65
SEQ ID No.: **262F**
   Sequence Type: AA
   Sequence Length: 77 AA 
Gly-Tyr-Lys-Val-Leu-Val-Leu-Asn-Pro-Ser-Val-Ala-Ala-Thr-Leu-
5 10 15
Gly-Phe-Gly-Ala-Tyr-Met-Ser-Lys-Ala-His-Gly-Ile-Asp-Pro-Asn-
20 25 30
Ile-Arg-Thr-Gly-Val-Arg-Thr-Ile-Thr-Thr-Gly-Ser-Pro-Ile-Thr-
35 40 45
Tyr-Ser-Thr-Tyr-Gly-Lys-Phe-Leu-Ala-Asp-Gly-Gly-Cys-Ser-Gly-
50 55 60
Gly-Ala-Tyr-Asp-Ile-Ile-Ile-Cys-Asp-Glu-Cys-His-Ser-Thr-Asp-
65 70 75
Ala-Thr
SEQ ID No.: **261A**
   Sequence Type: AA
   Sequence Length: 30 AA 
Pro-Phe-Tyr-Gly-Lys-Ala-Ile-Pro-Leu-Glu-Val-Ile-Lys-Gly-Gly-
                5                   10                  15
Arg-His-Leu-Ile-Phe-Cys-His-Ser-Lys-Lys-Lys-Cys-Asp-Glu-Leu
                20                  25                  30
SEQ ID No.: **261B**
   Sequence Type: AA
   Sequence Length: 40 AA 
Glu-Val-Ala-Leu-Ser-Thr-Thr-Gly-Glu-Ile-Pro-Phe-Tyr-Gly-Lys-
                    5                   10                  15
Ala-Ile-Pro-Leu-Glu-Val-Ile-Lys-Gly-Gly-Arg-His-Leu-Ile-Phe-
                   20                   25                  30
Cys-His-Ser-Lys-Lys-Lys-Cys-Asp-Glu-Leu
                   35                   40
SEQ ID No.: **261C**
   Sequence Type: AA
   Sequence Length: 50 AA 
Ser-Val-Thr-Val-Pro-His-Pro-Asn-Ile-Glu-Glu-Val-Ala-Leu-Ser-
                    5                   10                  15
Thr-Thr-Gly-Glu-Ile-Pro-Phe-Tyr-Gly-Lys-Ala-Ile-Pro-Leu-Glu-
                    20                  25                  30
Val-Ile-Lys-Gly-Gly-Arg-His-Leu-Ile-Phe-Cys-His-Ser-Lys-Lys-
                    35                  40                  45
Lys-Cys-Asp-Glu-Leu
                    50
SEQ ID No.: **261D**
   Sequence Type: AA
   Sequence Length: 73 AA 
Thr-Val-Leu-Asp-Gln-Ala-Glu-Thr-Ala-Gly-Ala-Arg-Leu-Val-Val-
5 10 15
Leu-Ala-Thr-Ala-Thr-Pro-Pro-Gly-Ser-Val-Thr-Val-Pro-His-Pro-
20 25 30
Asn-Ile-Glu-Glu-Val-Ala-Leu-Ser-Thr-Thr-Gly-Glu-Ile-Pro-Phe-
35 40 45
Tyr-Gly-Lys-Ala-Ile-Pro-Leu-Glu-Val-Ile-Lys-Gly-Gly-Arg-His-
50 55 60
Leu-Ile-Phe-Cys-His-Ser-Lys-Lys-Lys-Cys-Asp-Glu-Leu
65 70
SEQ ID No.: **261E**
   Sequence Type: AA
   Sequence Length: 97 AA 
Gly-Gly-Ala-Tyr-Asp-Ile-Ile-Ile-Cys-Asp-Glu-Cys-His-Ser-Thr-
5 10 15
Asp-Ala-Thr-Ser-Ile-Leu-Gly-Ile-Gly-Thr-Val-Leu-Asp-Gln-Ala-
20 25 30
Glu-Thr-Ala-Gly-Ala-Arg-Leu-Val-Val-Leu-Ala-Thr-Ala-Thr-Pro-
35 40 45
Pro-Gly-Ser-Val-Thr-Val-Pro-His-Pro-Asn-Ile-Glu-Glu-Val-Ala-
50 55 60
Leu-Ser-Thr-Thr-Gly-Glu-Ile-Pro-Phe-Tyr-Gly-Lys-Ala-Ile-Pro-
65 70 75
Leu-Glu-Val-Ile-Lys-Gly-Gly-Arg-His-Leu-Ile-Phe-Cys-His-Ser-
80 85 90
Lys-Lys-Lys-Cys-Asp-Glu-Leu
95
SEQ ID No.: **261F**
   Sequence Type: AA
   Sequence Length: 121 AA 
Arg-Thr-Ile-Thr-Thr-Gly-Ser-Pro-Ile-Thr-Tyr-Ser-Thr-Tyr-Gly-
5 10 15
Lys-Phe-Leu-Ala-Asp-Gly-Gly-Cys-Ser-Gly-Gly-Ala-Tyr-Asp-Ile-
20 25 30
Ile-Ile-Cys-Asp-Glu-Cys-His-Ser-Thr-Asp-Ala-Thr-Ser-Ile-Leu-
35 40 45
Gly-Ile-Gly-Thr-Val-Leu-Asp-Gln-Ala-Glu-Thr-Ala-Gly-Ala-Arg-
50 55 60
Leu-Val-Val-Leu-Ala-Thr-Ala-Thr-Pro-Pro-Gly-Ser-Val-Thr-Val-
65 70 75
Pro-His-Pro-Asn-Ile-Glu-Glu-Val-Ala-Leu-Ser-Thr-Thr-Gly-Glu-
80 85 90
Ile-Pro-Phe-Tyr-Gly-Lys-Ala-Ile-Pro-Leu-Glu-Val-Ile-Lys-Gly-
95 100 105
Gly-Arg-His-Leu-Ile-Phe-Cys-His-Ser-Lys-Lys-Lys-Cys-Asp-Glu-
110 115 120
Leu
SEQ ID No.: **279A (Pep 3)**
   Sequence Type: AA
   Sequence Length: 37 AA 
Gly-Cys-Ser-Gly-Gly-Ala-Tyr-Asp-Ile-Ile-Ile-Cys-Asp-Glu-Cys-
                5                   10                  15
His-Ser-Thr-Asp-Ala-Thr-Ser-Ile-Leu-Gly-Ile-Gly-Thr-Val-Leu-
                20                  25                  30
Asp-Gln-Ala-Glu-Thr-Ala-Gly
                35
SEQ ID No.: **279B**
   Sequence Type: AA
   Sequence Length: 42 AA 
Phe-Leu-Ala-Asp-Gly-Gly-Cys-Ser-Gly-Gly-Ala-Tyr-Asp-Ile-Ile-
5 10 15
Ile-Cys-Asp-Glu-Cys-His-Ser-Thr-Asp-Ala-Thr-Ser-Ile-Leu-Gly-
20 25 30
Ile-Gly-Thr-Val-Leu-Asp-Gln-Ala-Glu-Thr-Ala-Gly
35 40
SEQ ID No.: **279E**
   Sequence Type: AA
   Sequence Length: 58 AA 
Arg-Thr-Ile-Thr-Thr-Gly-Ser-Pro-Ile-Thr-Tyr-Ser-Thr-Tyr-Gly-
                5                   10                  15
Lys-Phe-Leu-Ala-Asp-Gly-Gly-Cys-Ser-Gly-Gly-Ala-Tyr-Asp-Ile-
                20                  25                  30
Ile-Ile-Cys-Asp-Glu-Cys-His-Ser-Thr-Asp-Ala-Thr-Ser-Ile-Leu-
                35                  40                  45
Gly-Ile-Gly-Thr-Val-Leu-Asp-Gln-Ala-Glu-Thr-Ala-Gly
                50                  55
SEQ ID No.: **255A**
   Sequence Type: AA
   Sequence Length: 35 AA 
Thr-Asn-Asp-Cys-Pro-Asn-Ser-Ser-Ile-Val-Tyr-Glu-Ala-His-Asp-
5 10 15
Ala-Ile-Leu-His-Thr-Pro-Gly-Cys-Val-Pro-Cys-Val-Arg-Glu-Gly-
20 25 30
Asn-Val-Ser-Arg-Cys
35
SEQ ID No.: **255B**
   Sequence Type: AA
   Sequence Length: 45 AA 
Val-Arg-Asn-Ser-Thr-Gly-Leu-Tyr-His-Val-Thr-Asn-Asp-Cys-Pro-
5 10 15
Asn-Ser-Ser-Ile-Val-Tyr-Glu-Ala-His-Asp-Ala-Ile-Leu-His-Thr-
20 25 30
Pro-Gly-Cys-Val-Pro-Cys-Val-Arg-Glu-Gly-Asn-Val-Ser-Arg-Cys-
35 40 45
SEQ ID No.: **255C (Pep 7)**
   Sequence Type: AA
   Sequence Length: 55 AA 
Cys-Leu-Thr-Val-Pro-Ala-Ser-Ala-Tyr-Gln-Val-Arg-Asn-Ser-Thr-
5 10 15
Gly-Leu-Tyr-His-Val-Thr-Asn-Asp-Cys-Pro-Asn-Ser-Ser-Ile-Val-
20 25 30
Tyr-Glu-Ala-His-Asp-Ala-Ile-Leu-His-Thr-Pro-Gly-Cys-Val-Pro-
35 40 45
Cys-Val-Arg-Glu-Gly-Asn-Val-Ser-Arg-Cys
50 55
SEQ ID No.: **244A**
   Sequence Type: AA
   Sequence Length: 35 AA 
Cys-Trp-Val-Ala-Met-Thr-Pro-Thr-Val-Ala-Thr-Arg-Asp-Gly-Lys-
                5                   10                  15
Leu-Pro-Ala-Thr-Gln-Leu-Arg-Arg-His-Ile-Asp-Leu-Leu-Val-Gly-
                20                  25                  30
Ser-Ala-Thr-Leu-Cys
                35
SEQ ID No.: **244B**
   Sequence Type: AA
   Sequence Length: 44 AA 
Cys-Val-Arg-Glu-Gly-Asn-Val-Ser-Arg-Cys-Trp-Val-Ala-Met-Thr-
5 10 15
Pro-Thr-Val-Ala-Thr-Arg-Asp-Gly-Lys-Leu-Pro-Ala-Thr-Gln-Leu-
20 25 30
Arg-Arg-His-Ile-Asp-Leu-Leu-Val-Gly-Ser-Ala-Thr-Leu-Cys
35 40
SEQ ID No.: **254A**
   Sequence Type: AA
   Sequence Length: 30 AA 
Thr-Gln-Gly-Cys-Asn-Cys-Ser-Ile-Tyr-Pro-Gly-His-Ile-Thr-Gly-
                5                   10                  15
His-Arg-Met-Ala-Trp-Asp-Met-Met-Met-Asn-Trp-Ser-Pro-Thr-Ala
                20                  25                  30
SEQ ID No.: **254B (Pep 8)**
   Sequence Type: AA
   Sequence Length: 40 AA 
Phe-Thr-Phe-Ser-Pro-Arg-Arg-His-Trp-Thr-Thr-Gln-Gly-Cys-Asn-
5 10 15
Cys-Ser-Ile-Tyr-Pro-Gly-His-Ile-Thr-Gly-His-Arg-Met-Ala-Trp-
20 25 30
Asp-Met-Met-Met-Asn-Trp-Ser-Pro-Thr-Ala
35 40
SEQ ID No.: **254C**
   Sequence Type: AA
   Sequence Length: 50 AA 
Cys-Gly-Ser-Val-Phe-Leu-Ile-Gly-Gln-Leu-Phe-Thr-Phe-Ser-Pro-
                5                   10                  15
Arg-Arg-His-Trp-Thr-Thr-Gln-Gly-Cys-Asn-Cys-Ser-Ile-Tyr-Pro-
                20                  25                  30
Gly-His-Ile-Thr-Gly-His-Arg-Met-Ala-Trp-Asp-Met-Met-Met-Asn-
                35                  40                  45
Trp-Ser-Pro-Thr-Ala
                50
SEQ ID No.: **248A**
   Sequence Type: AA
   Sequence Length: 25 AA 
Asp-Met-Ile-Ala-Gly-Ala-His-Trp-Gly-Val-Leu-Ala-Gly-Ile-Ala-
                5                   10                  15
Tyr-Phe-Ser-Met-Val-Gly-Asn-Trp-Ala-Lys
                20                  25
SEQ ID No.: **248B**
   Sequence Type: AA
   Sequence Length: 35 AA 
Gln-Leu-Leu-Arg-Ile-Pro-Gln-Ala-Ile-Leu-Asp-Met-Ile-Ala-Gly-
                5                   10                  15
Ala-His-Trp-Gly-Val-Leu-Ala-Gly-Ile-Ala-Tyr-Phe-Ser-Met-Val-
                20                  25                  30
Gly-Asn-Trp-Ala-Lys
                35
SEQ ID No.: **248C**
   Sequence Type: AA
   Sequence Length: 40 AA 
Ala-Leu-Val-Met-Ala-Gln-Leu-Leu-Arg-Ile-Pro-Gln-Ala-Ile-Leu-
                5                   10                  15
Asp-Met-IleAla-Gly-Ala-His-Trp-Gly-Val-Leu-Ala-Gly-Ile-Ala-
               20                  25                  30
Tyr-Phe-Ser-Met-Val-Gly-Asn-Trp-Ala-Lys
               35                  40
SEQ ID No.: **247A**
   Sequence Type: AA
   Sequence Length: 25 AA 
Gln-Ala-Ala-Arg-Ala-Met-Ser-Gly-Leu-Val-Ser-Leu-Phe-Thr-Pro-
                    5                        10                        15
Gly-Ala-Lys-Gln-Asn-Ile-Gln-Leu-Ile-Asn
                    20                       25
SEQ ID No.: **247B (Pep 9)**
   Sequence Type: AA
   Sequence Length: 35 AA 
Val-Asp-Ala-Glu-Thr-Ile-Val-Ser-Gly-Gly-Gln-Ala-Ala-Arg-Ala-
                5                   10                  15
Met-Ser-Gly-Leu-Val-Ser-Leu-Phe-Thr-Pro-Gly-Ala-Lys-Gln-Asn-
                20                  25                  30
Ile-Gln-Leu-Ile-Asn
                35
SEQ ID No.: **247C**
   Sequence Type: AA
   Sequence Length: 45AA 
Val-Leu-Val-Val-Leu-Leu-Leu-Phe-Ala-Gly-Val-Asp-Ala-Glu-Thr-
                5                   10                  15
Ile-Val-Ser-Gly-Gly-Gln-Ala-Ala-Arg-Ala-Met-Ser-Gly-Leu-Val-
                20                  25                  30
Ser-Leu-Phe-Thr-Pro-Gly-Ala-Lys-Gln-Asn-Ile-Gln-Leu-Ile-Asn
                35                  40                  45
SEQ ID No.: **247D**
   Sequence Type: AA
   Sequence Length: 55 AA 
Tyr-Phe-Ser-Met-Val-Gly-Asn-Trp-Ala-Lys-Val-Leu-Val-Val-Leu-
                5                   10                  15
Leu-Leu-Phe-Ala-Gly-Val-Asp-Ala-Glu-Thr-Ile-Val-Ser-Gly-Gly-
                20                  25                  30
Gln-Ala-Ala-Arg-Ala-Met-Ser-Gly-Leu-Val-Ser-Leu-Phe-Thr-Pro-
                35                  40                  45
Gly-Ala-Lys-Gln-Asn-Ile-Gln-Leu-Ile-Asn
                50                  55
SEQ ID No.: **247E**
   Sequence Type: AA
   Sequence Length: 60 AA 
Leu-Ala-Gly-Ile-Ala-Tyr-Phe-Ser-Met-Val-Gly-Asn-Trp-Ala-Lys-
                5                   10                  15
Val-Leu-Val-Val-Leu-Leu-Leu-Phe-Ala-Gly-Val-Asp-Ala-Glu-Thr-
                20                  25                  30
Ile-Val-Ser-Gly-Gly-Gln-Ala-Ala-Arg-Ala-Met-Ser-Gly-Leu-Val-
                35                  40                  45
Ser-Leu-Phe-Thr-Pro-Gly-Ala-Lys-Gln-Asn-Ile-Gln-Leu-Ile-Asn
                50                  55                  60
SEQ ID No.: **246A**
   Sequence Type: AA
   Sequence Length: 25 AA 
Thr-Gly-Trp-Leu-Ala-Gly-Leu-Ile-Tyr-Gln-His-Lys-Phe-Asn-Ser-
5 10 15
Ser-Gly-Cys-Pro-Glu-Arg-Leu-Ala-Ser-Cys
20 25
SEQ ID No.: **246B**
   Sequence Type: AA
   Sequence Length: 31 AA 
Cys-Asn-Glu-Ser-Leu-Asn-Thr-Gly-Trp-Leu-Ala-Gly-Leu-Ile-Tyr-
5 10 15
Gln-His-Lys-Phe-Asn-Ser-Ser-Gly-Cys-Pro-Glu-Arg-Leu-Ala-Ser-
20 25 30
Cys
SEQ ID No.: **246C**
   Sequence Type: AA
   Sequence Length: 38 AA 
Ile-Asn-Ser-Thr-Ala-Leu-Asn-Cys-Asn-Glu-Ser-Leu-Asn-Thr-Gly-
                5                   10                  15
Trp-Leu-Ala-Gly-Leu-Ile-Tyr-Gln-His-Lys-Phe-Asn-Ser-Ser-Gly-
                20                  25                  30
Cys-Pro-Glu-Arg-Leu-Ala-Ser-Cys
                35
SEQ ID No.: **246D (Pep 10)**
   Sequence Type: AA
   Sequence Length: 40 AA 
Trp-His-Ile-Asn-Ser-Thr-Ala-Leu-Asn-Cys-Asn-Glu-Ser-Leu-Asn-
5 10 15
Thr-Gly-Trp-Leu-Ala-Gly-Leu-Ile-Tyr-Gln-His-Lys-Phe-Asn-Ser-
20 25 30
Ser-Gly-Cys-Pro-Glu-Arg-Leu-Ala-Ser-Cys
35 40
SEQ ID No.: **246E**
   Sequence Type: AA
   Sequence Length: 52 AA 
Lys-Gln-Asn-Ile-Gln-Leu-Ile-Asn-Thr-Asn-Gly-Ser-Trp-His-Ile-
                5                   10                  15
Asn-Ser-Thr-Ala-Leu-Asn-Cys-Asn-Glu-Ser-Leu-Asn-Thr-Gly-Trp-
                20                  25                  30
Leu-Ala-Gly-Leu-Ile-Tyr-Gln-His-Lys-Phe-Asn-Ser-Ser-Gly-Cys-
                35                  40                  45
Pro-Glu-Arg-Leu-Ala-Ser-Cys
                50
SEQ ID No.: **314A (Pep 16)**
   Sequence Length: 30 AA 
Gly-Ser-Trp-Leu-Arg-Asp-Ile-Trp-Asp-Trp-Ile-Cys-Glu-Val-Leu-
                    5                   10                  15
Ser-Asp-Phe-Lys-Thr-Trp-Leu-Lys-Ala-Lys-Leu-Met-Pro-Gln-Leu
                    20                  25                  30
SEQ ID No.: **314B**
   Sequence Type: AA
   Sequence Length: 38 AA 
Ser-Glu-Cys-Thr-Thr-Pro-Cys-Ser-Gly-Ser-Trp-Leu-Arg-Asp-Ile-
                5                   10                  15
Trp-Asp-Trp-Ile-Cys-Glu-Val-Leu-Ser-Asp-Phe-Lys-Thr-Trp-Leu-
                20                  25                  30
Lys-Ala-Lys-Leu-Met-Pro-Gln-Leu
                35
SEQ ID No.: **314C**
   Sequence Type: AA
   Sequence Length: 47 AA 
Leu-Arg-Arg-Leu-His-Gln-Trp-Ile-Ser-Ser-Glu-Cys-Thr-Thr-Pro-
5 10 15
Cys-Ser-Gly-Ser-Trp-Leu-Arg-Asp-Ile-Trp-Asp-Trp-Ile-Cys-Glu-
20 25 30
Val-Leu-Ser-Asp-Phe-Lys-Thr-Trp-Leu-Lys-Ala-Lys-Leu-Met-Pro-
35 40 45
Gln-Leu
SEQ ID No.: **312A**
   Sequence Type: AA
   Sequence Length: 22 AA 
Asp-Phe-His-Tyr-Val-Thr-Gly-Met-Thr-Thr-Asp-Asn-Leu-Lys-Cys-
                5                   10                  15
Pro-Cys-Gln-Val-Pro-Ser-Pro
                20
SEQ ID No.: **312B (Pep 15)**
   Sequence Type: AA
   Sequence Length: 32 AA 
Glu-Glu-Tyr-Val-Glu-Ile-Arg-Gln-Val-Gly-Asp-Phe-His-Tyr-Val-
                5                   10                  15
Thr-Gly-Met-Thr-Thr-Asp-Asn-Leu-Lys-Cys-Pro-Cys-Gln-Val-Pro-
                20                  25                  30
Ser-Pro
SEQ ID No.: **312C**
   Sequence Type: AA
   Sequence Length: 38 AA 
Leu-Trp-Arg-Val-Ser-Ala-Glu-Glu-Tyr-Val-Glu-Ile-Arg-Gln-Val-
                5                   10                  15
Gly-Asp-Phe-His-Tyr-Val-Thr-Gly-Met-Thr-Thr-Asp-Asn-Leu-Lys-
                20                  25                  30
Cys-Pro-Cys-Gln-Val-Pro-Ser-Pro
                35
SEQ ID No.: **311A (Pep14)**
   Sequence Type: AA
   Sequence Length: 31 AA 
Cys-Lys-Pro-Leu-Leu-Arg-Glu-Glu-Val-Ser-Phe-Arg-Val-Gly-Leu-
                5                   10                  15
His-Glu-Tyr-Pro-Val-Gly-Ser-Gln-Leu-Pro-Cys-Glu-Pro-Glu-Pro-
                20                  25                  30
Asp
SEQ ID No.: **311B**
   Sequence Type: AA
   Sequence Length: 42 AA 
Asp-Gly-Val-Arg-Leu-His-Arg-Phe-Ala-Pro-Pro-Cys-Lys-Pro-Leu-
5 10 15
Leu-Arg-Glu-Glu-Val-Ser-Phe-Arg-Val-Gly-Leu-His-Glu-Tyr-Pro-
20 25 30
Val-Gly-Ser-Gln-Leu-Pro-Cys-Glu-Pro-Glu-Pro-Asp
35 40
SEQ ID No.: **311C**
   Sequence Type: AA
   Sequence Length: 54 AA 
Cys-Gln-Val-Pro-Ser-Pro-Glu-Phe-Phe-Thr-Glu-Leu-Asp-Gly-Val-
5 10 15
Arg-Leu-His-Arg-Phe-Ala-Pro-Pro-Cys-Lys-Pro-Leu-Leu-Arg-Glu-
20 25 30
Glu-Val-Ser-Phe-Arg-Val-Gly-Leu-His-Glu-Tyr-Pro-Val-Gly-Ser-
35 40 45
Gln-Leu-Pro-Cys-Glu-Pro-Glu-Pro-Asp
50
SEQ ID No.: **260A**
   Sequence Type: AA
   Sequence Length: 23 AA 
Ser-Ser-Ser-Ala-Ser-Gln-Leu-Ser-Ala-Pro-Ser-Leu-Lys-Ala-Thr-
                5                   10                  15
Cys-Thr-Ala-Asn-His-Asp-Ser-Pro
                20
SEQ ID No.: **260B**
   Sequence Typet AA
   Sequence Length: 35 AA 
Arg-Arg-Leu-Ala-Arg-Gly-Ser-Pro-Pro-Ser-Val-Ala-Ser-Ser-Ser-
5 10 15
Ala-Ser-Gln-Leu-Ser-Ala-Pro-Ser-Leu-Lys-Ala-Thr-Cys-Thr-Ala-
20 25 30
Asn-His-Asp-Ser-Pro
35
SEQ ID No.: **260C (Pep 4)**
   Sequence Type: AA
   Sequence Length: 46 AA 
Asp-Pro-Ser-His-Ile-Thr-Ala-Glu-Ala-Ala-Gly-Arg-Arg-Leu-Ala-
                5                   10                  15
Arg-Gly-Ser-Pro-Pro-Ser-Val-Ala-Ser-Ser-Ser-Ala-Ser-Gln-Leu-
                20                  25                  30
Ser-Ala-Pro-Ser-Leu-Lys-Ala-Thr-Cys-Thr-Ala-Asn-His-Asp-Ser-
                35                  40                  45
Pro
SEQ ID No.: **259B**
   Sequence Type: AA
   Sequence Length: 35 AA 
Leu-Trp-Arg-Gln-Glu-Met-Gly-Gly-Asn-Ile-Thr-Arg-Val-Glu-Ser-
                5                   10                  15
Glu-Asn-Lys-Val-Val-Ile-Leu-Asp-Ser-Phe-Asp-Pro-Leu-Val-Ala-
                20                  25                  30
Glu-Glu-Asp-Glu-Arg
                35
SEQ ID No.: **259C (Pep 5)**
   Sequence Type: AA
   Sequence Length: 44 AA 
Asp-Ala-Glu-Leu-Ile-Glu-Ala-Asn-Leu-Leu-Trp-Arg-Gln-Glu-Met-
5 10 15
Gly-Gly-Asn-Ile-Thr-Arg-Val-Glu-Ser-Glu-Asn-Lys-Val-Val-Ile-
20 25 30
Leu-Asp-Ser-Phe-Asp-Pro-Leu-Val-Ala-Glu-Glu-Asp-Glu-Arg
35 40
SEQ ID No.: **259D**
   Sequence Type: AA
   Sequence Length: 50 AA 
Ala-Asn-His-Asp-Ser-Pro-Asp-Ala-Glu-Leu-Ile-Glu-Ala-Asn-Leu-
                5                   10                  15
Leu-Trp-Arg-Gln-Glu-Met-Gly-Gly-Asn-Ile-Thr-Arg-Val-Glu-Ser-
                20                  25                  30
Glu-Asn-Lys-Val-Val-Ile-Leu-Asp-Ser-Phe-Asp-Pro-Leu-Val-Ala-
                35                  40                  45
Glu-Glu-Asp-Glu-Arg
                50
SEQ ID No.: **259E (Pep 12)**
   Sequence Type: AA
   Sequence Length: 55 AA 
Lys-Ala-Thr-Cys-Thr-Ala-Asn-His-Asp-Ser-Pro-Asp-Ala-Glu-Leu-
5 10 15
Ile-Glu-Ala-Asn-Leu-Leu-Trp-Arg-Gln-Glu-Met-Gly-Gly-Asn-Ile-
20 25 30
Thr-Arg-Val-Glu-Ser-Glu-Asn-Lys-Val-Val-Ile-Leu-Asp-Ser-Phe-
35 40 45
Asp-Pro-Leu-Val-Ala-Glu-Glu-Asp-Glu-Arg
50 55
SEQ ID No.: **310A**
   Sequence Type: AA
   Sequence Length: 26 AA 
Ser-Phe-Asp-Pro-Leu-Val-Ala-Glu-Glu-Asp-Glu-Arg-Glu-Ile-Ser-
                5                   10                  15
Val-Pro-Ala-Glu-Ile-Leu-Arg-Lys-Ser-Arg-Arg
                20                  25
SEQ ID No.: **310B**
   Sequence Type: AA
   Sequence Length: 35 AA 
Ser-Glu-Asn-Lys-Val-Val-Ile-Leu-Asp-Ser-Phe-Asp-Pro-Leu-Val-
                5                   10                  15
Ala-Glu-Glu-Asp-Glu-Arg-Glu-Ile-Ser-Val-Pro-Ala-Glu-Ile-Leu-
                20                  25                  30
Arg-Lys-Ser-Arg-Arg
                35
SEQ ID No.: **310C (Pep 13)**
   Sequence Type: AA
   Sequence Length: 47 AA 
Arg-Gln-Glu-Met-Gly-Gly-Asn-Ile-Thr-Arg-Val-Glu-Ser-Glu-Asn-
5 10 15
Lys-Val-Val-Ile-Leu-Asp-Ser-Phe-Asp-Pro-Leu-Val-Ala-Glu-Glu-
20 25 30
Asp-Glu-Arg-Glu-Ile-Ser-Val-Pro-Ala-Glu-Ile-Leu-Arg-Lys-Ser-
35 40 45
Arg-Arg
SEQ ID No.: **309A**
   Sequence Type: AA
   Sequence Lenghh: 27 AA 
Tyr-Glu-Pro-Pro-Val-Val-His-Gly-Cys-Pro-Leu-Pro-Pro-Pro-Lys-
                5                   10                  15
Ser-Pro-Pro-Val-Pro-Pro-Pro-Arg-Lys-Lys-Arg-Thr
                20                  25
SEQ ID No.: **309B**
   Sequence Type: AA
   Sequence Lentgh: 35 AA 
Val-Glu-Thr-Trp-Lys-Lys-Pro-Asp-Tyr-Glu-Pro-Pro-Val-Val-His-
5 10 15
Gly-Cys-Pro-Leu-Pro-Pro-Pro-Lys-Ser-Pro-Pro-Val-Pro-Pro-Pro-
20 25 30
Arg-Lys-Lys-Arg-Thr
35
SEQ ID No.: **309C**
   Sequence Type: AA
   Sequence Length: 44 AA 
Ala-Arg-Pro-Asp-Tyr-Asn-Pro-Pro-Leu-Val-Glu-Thr-Trp-Lys-Lys-
                5                   10                  15
Pro-Asp-Tyr-Glu-Pro-Pro-Val-Val-His-Gly-Cys-Pro-Leu-Pro-Pro-
                20                  25                  30
Pro-Lys-Ser-Pro-Pro-Val-Pro-Pro-Pro-Arg-Lys-Lys-Arg-Thr
                35                  40
SEQ ID No.: **309D (Pep 11)**
   Sequence Type: AA
   Sequence Length: 60 AA 
Glu-Ile-Leu-Arg-Lys-Ser-Arg-Arg-Phe-Ala-Gln-Ala-Leu-Pro-Val-
5 10 15
Trp-Ala-Arg-Pro-Asp-Tyr-Asn-Pro-Pro-Leu-Val-Glu-Thr-Trp-Lys-
20 25 30
Lys-Pro-Asp-Tyr-Glu-Pro-Pro-Val-Val-His-Gly-Cys-Pro-Leu-Pro-
35 40 45
Pro-Pro-Lys-Ser-Pro-Pro-Val-Pro-Pro-Pro-Arg-Lys-Lys-Arg-Thr
50 55 60
SEQ ID No.: **309E**
   Sequence Type: AA
   Sequence Length: 72 AA 
Ala-Glu-Glu-Asp-Glu-Arg-Glu-Ile-Ser-Val-Pro-Ala-Glu-Ile-Leu-
5 10 15
Arg-Lys-Ser-Arg-Arg-Phe-Ala-Gln-Ala-Leu-Pro-Val-Trp-Ala-Arg-
20 25 30
Pro-Asp-Tyr-Asn-Pro-Pro-Leu-Val-Glu-Thr-Trp-Lys-Lys-Pro-Asp-
35 40 45
Tyr-Glu-Pro-Pro-Val-Val-His-Gly-Cys-Pro-Leu-Pro-Pro-Pro-Lys-
50 55 60
Ser-Pro-Pro-Val-Pro-Pro-Pro-Arg-Lys-Lys-Arg-Thr
65 70
SEQ ID No.: **Pep 6**
   Sequence Type: AA
   Sequence Length: 37 AA 
Asp-Pro-Gln-Ala-Arg-Val-Ala-Ile-Lys-Ser-Leu-Thr-Glu-Arg-Leu-
                5                   10                  15
Thr-Val-Gly-Gly-Pro-Leu-Thr-Asn-Ser-Arg-Gly-Glu-Asn-Cys-Gly-
                20                  25                  30
Tyr-Arg-Arg-Cys-Arg-Ala-Ser
                35
SEQ ID No.: **Pep 17**
   Sequence Type: AA
   Sequence Length: 45 AA 
Gly-Pro-Ala-Asp-Gly-Met-Val-Ser-Lys-Gly-Trp-Arg-Leu-Leu-Ala-
                5                   10                  15
Pro-Ile-Thr-Ala-Tyr-Ala-Gln-Gln-Thr-Arg-Gly-Leu-Leu-Gly-Cys-
                20                  25                  30
Ile-Ile-Thr-Ser-Leu-Thr-Gly-Arg-Asp-Lys-Asn-Gln-Val-Glu-Gly
                35                  40                  45
SEQ ID No.: **Pep 18**
   Sequence Type: AA
   Sequence Length: 39 AA 
Glu-Ile-Pro-Phe-Tyr-Gly-Lys-Ala-Ile-Pro-Leu-Glu-Val-Ile-Lys-
                5                   10                  15
Gly-Gly-Arg-His-Leu-Ile-Phe-Cys-His-Ser-Lys-Lys-Lys-Cys-Asp-
                20                  25                  30
Glu-Leu-Ala-Ala-Lys-Leu-Val-Ala-Leu
                35
SEQ ID No.: **Pep 19**
   Sequence Type: AA
   Sequence Length: 44 AA 
Cys-Val-Arg-Glu-Gly-Asn-Val-Ser-Arg-Cys-Trp-Val-Ala-Met-Thr-
                5                   10                  15
Pro-Thr-Val-Ala-Thr-Arg-Asp-Gly-Lys-Leu-Pro-Ala-Thr-Gln-Leu-
                20                  25                  30
Arg-Arg-His-Ile-Asp-Leu-Leu-Val-Gly-Ser-Ala-Thr-Leu-Cys
                35                  40
SEQ ID No.: **VIIIE**
   Sequence Type: AA
   Sequence Length: 61 AA 
Ser-Thr-Ile-Pro-Lys-Pro-Gln-Arg-Lys-Thr-Lys-Arg-Asn-Thr-Asn-
                    5                        10                        15
Arg-Arg-Pro-Gln-Asp-Val-Lys-Phe-Pro-Gly-Gly-Gly-Gln-Ile-Val-
                    20                       25                        30
Gly-Gly-Val-Tyr-Leu-Leu-Pro-Arg-Arg-Gly-Pro-Arg-Leu-Gly-Val-
                    35                       40                        45
Arg-Ala-Thr-Arg-Lys-Thr-Ser-Glu-Arg-Ser-Gln-Pro-Arg-Gly-Arg-
                    50                       55                        60
Arg
SEQ ID No.: **IIH**
   Sequence Type: AA
   Sequence Length: 47 AA 
Ser-Gly-Lys-Pro-Ala-Ile-Ile-Pro-Asp-Arg-Glu-Val-Leu-Tyr-Arg-
                5                   10                  15
Glu-Phe-Asp-Glu-Met-Glu-Glu-Cys-Ser-Gln-His-Leu-Pro-Tyr-Ile-
                20                  25                  30
Glu-Gln-Gly-Met-Met-Leu-Ala-Glu-Gln-Phe-Lys-Gln-Lys-Ala-Leu-
                35                  40                  45
Gly-Leu
SEQ ID No.: **V**
   Sequence Type: AA
   Sequence Length: 40 AA 
Lys-Gln-Lys-Ala-Leu-Gly-Leu-Leu-Gln-Thr-Ala-Ser-Arg-Gln-Ala-
5 10 15
Glu-Val-Ile-Ala-Pro-Ala-Val-Gln-Thr-Asn-Trp-Gln-Lys-Leu-Glu-
20 25 30
Thr-Phe-Trp-Ala-Lys-His-Met-Trp-Asn-Phe
35 40
SEQ ID No.: **Pep XX**
   Sequence Type: AA
   [Peptide]16 Lys₈ Lys₄ Lys₂ Lys-Y decahexyl peptide
SEQ ID No.: **Pep XXI**
   Sequence Type: AA
   [Peptide]₈ Lys₄ Lys₂ Lys-Y octameric peptide
SEQ ID No.: **Pep XXII**
   Sequence Type: AA
   [Peptide]₄ Lys₂ Lys-Y tetrameric peptide
SEQ ID No.: **Pep XXIII**
   Sequence Type: AA
   [Peptide]₂ Lys-Y dimeric peptide
wherein the peptide in Pep XX, Pep XXI, Pep XXII and Pep XIII is selected from the group consisting of (a) to (j):

(a)    Cys-Leu-Thr-Ile-Pro-Ala-Ser-Ala-Tyr-Glu-Val-Arg-Asn-Val-Ser-
                        5                        10                        15
       Gly-Ile-Tyr-His-Val-Thr-Asn-Asp-Cys-Ser-Asn-Ser-Ser-Ile-Val-
                       20                        25                        30
       Tyr-Glu-Ala-Ala-Asp-Val-Ile-Met-His-Ala-Pro-Gly-Cys-Val-Pro-
                       35                        40                        45
       Cys-Val-Arg-Glu-Asn-Asn-Ser-Ser-Arg-Cys
                       50                        55

(b)    Cys-Ile-Thr-Thr-Pro-Val-Ser-Ala-Ala-Glu-Val-Lys-Asn-Ile-Ser-
                       5                   10                  15
       Thr-Gly-Tyr-Met-Val-Thr-Asn-Asp-Cys-Thr-Asn-Asp-Ser-Ile-Thr-
                       20                  25                  30
       Trp-Gln-Leu-Gln-Ala-Ala-Val-Leu-His-Val-Pro-Gly-Cys-Val-Pro-
                       35                  40                  45
       Cys-Glu-Lys-Val-Gly-Asn-Thr-Ser-Arg-Cys
                       50                  55

(c) Cys-Val-Thr-Val-Pro-Val-Ser-Ala-Val-Glu-Val-Arg-Asn-Ile-Ser-
5 10 15
Ser-Ser-Tyr-Tyr-Ala-Thr-Asn-Asp-Cys-Ser-Asn-Asn-Ser-Ile-Thr-
20 25 30
Trp-Gln-Leu-Thr-Asn-Ala-Val-Leu-His-Leu-Pro-Gly-Cys-Val-Pro-
35 40 45
Cys-Glu-Asn-Asp-Asn-Gly-Thr-Leu-Arg-Cys
50 55

(d)    Phe-Thr-Phe-Ser-Pro-Arg-Arg-His-Glu-Thr-Val-Gln-Asp-Cys-Asn-
                       5                   10                  15
       Cys-Ser-Ile-Tyr-Pro-Gly-His-Val-Ser-Gly-His-Arg-Met-Ala-Trp-
                      20                   25                  30
       Asp-Met-Met-Met-Asn-Trp-Ser-Pro-Thr-Ala
                      35                   40

(e)    Phe-Ile-Val-Ser-Pro-Gln-His-His-His-Phe-Val-Gln-Asp-Cys-Asn-
                          5                   10                  15
       Cys-Ser-Ile-Tyr-Pro-Gly-Thr-Ile-Thr-Gly-His-Arg-Met-Ala-Trp-
                          20                  25                  30
       Asp-Met-Met-Met-Asn-Trp-Ser-Pro-Thr-Ala
                          35                  40

(f)   Phe-Ile-Ile-Ser-Pro-Glu-Arg-Asn-Phe-Thr-Gln-Glu-Cys-Asn-Cys-
                      5                        10                       15
      Ser-Ile-Tyr-Gln-Gly-His-Ile-Thr-Gly-His-Arg-Met-Ala-Trp-Asp-
                      20                       25                       30
      Met-Met-Leu-Asn-Trp-Ser-Pro-Thr-Leu
                      35

(g) Cys-Val-Arg-Glu-Gly-Asn-Val-Ser-Arg-Cys-Trp-Val-Ala-Met-Thr-
5 10 15
Pro-Thr-Val-Ala-Thr-Arg-Asp-Gly-Lys-Leu-Pro-Ala-Thr-Gln-Leu-
20 25 30
Arg-Arg-His-Ile-Asp-Leu-Leu-Val-Gly-Ser-Ala-Thr-Leu-Cys
35 40

(h)   Cys-Val-Arg-Glu-Asn-Asn-Ser-Ser-Arg-Cys-Trp-Val-Ala-Leu-Thr-
                      5                   10                  15
      Pro-Thr-Leu-Ala-Ala-Arg-Asn-Ala-Ser-Val-Pro-Thr-Thr-Thr-Leu-
                     20                   25                  30
      Arg-Arg-His-Val-Asp-Leu-Leu-Val-Gly-Thr-Ala-Ala-Phe-Cys
                     35                   40

(i) Cys-Glu-Lys-Val-Gly-Asn-Thr-Ser-Arg-Cys-Trp-Ile-Pro-Val-Ser-
5 10 15
Pro-Asn-Val-Ala-Val-Gln-Gln-Pro-Gly-Ala-Leu-Thr-Gln-Gly-Leu-
20 25 30
Arg-Thr-His-Ile-Asp-Met-Val-Val-Met-Ser-Ala-Thr-Leu-Cys
35 40

(j)   Cys-Glu-Asn-Asp-Asn-Gly-Thr-Leu-Arg-Cys-Trp-Ile-Gln-Val-Thr-
                      5                   10                  15
      Pro-Asn-Val-Ala-Val-Lys-His-Arg-Gly-Ala-Leu-Thr-His-Asn-Leu-
                      20                  25                  30
      Arg-Thr-His-Val-Asp-Met-Ile-Val-Met-Ala-Ala-Thr-Val-Cys
                      35                  40

## Claims

1. A peptide composition comprising a peptide having an amino acid sequence: 
Glu-Ile-Leu-Arg-Lys-Ser-Arg-Arg-Phe-Ala-Gln-Ala-Leu-
Pro-Val-Trp-Ala-Arg-Pro-Asp-Tyr-Asn-Pro-Pro-Leu-Val-
Glu-Thr-Trp-Lys-Lys-Pro-Asp-Tyr-Glu-Pro-Pro-Val-Val-
His-Gly-Cys-Pro-Leu-Pro-Pro-Pro-Lys-Ser-Pro-Pro-Val-
Pro-Pro-Pro-Arg-Lys-Lys-Arg-Thr-X;
Pep11
 wherein X is -OH or -NH₂, or
an analogue peptide of the above peptide having an amino acid sequence from a strain/isolate of HCV in a region corresponding to the peptide and having specific immunoreactivity to antibodies to HCV,
a conjugate of the above peptide or said analogue peptide with a carrier protein, the conjugate having specific immunoreactivity to antibodies to HCV, and
a polymer of the above peptide, or said analogue peptide having specific immunoreactivity to antibodies to HCV said polymer being in dimeric, tetrameric, octameric and decahexyl forms of the peptide and said polymer being synthesised on a branching poly-L-lysine resin which can be Lys₈; Lys₄; Lys₂; Lys, Lys₄; Lys₂; Lys, Lys₂; Lys, Lys.

2. A peptide composition comprising a mixture of peptides VIIIE and pep 11 wherein peptide VIIIE is: 
Ser-Thr-Ile-Pro-Lys-Pro-Gln-Arg-Lys-Thr-Lys-Arg-Asn-
Thr-Asn-Arg-Arg-Pro-Gln-Asp-Val-Lys-Phe-Pro-Gly-Gly-
Gly-Gln-Ile-Val-Gly-Gly-Val-Tyr-Leu-Leu-Pro-Arg-Arg-
Gly-Pro-Arg-Leu-Gly-Val-Arg-Ala-Thr-Arg-Lys-Thr-Ser-
Glu-Arg-Ser-Gln-Pro-Arg-Gly-Arg-Arg-X;
(VIIIE)
 and pep11 is: 
Glu-Ile-Leu-Arg-Lys-Ser-Arg-Arg-Phe-Ala-Gln-Ala-Leu-
Pro-Val-Trp-Ala-Arg-Pro-Asp-Tyr-Asn-Pro-Pro-Leu-Val-
Glu-Thr-Trp-Lys-Lys-Pro-Asp-Tyr-Glu-Pro-Pro-Val-Val-
His-Gly-Cys-Pro-Leu-Pro-Pro-Pro-Lys-Ser-Pro-Pro-Val-
Pro-Pro-Pro-Arg-Lys-Lys-Arg-Thr-X;
Pep11
 wherein X is -OH or -NH₂, or
an analogue peptide of the above peptide having an amino acid sequence from a strain/isolate of HCV in a region corresponding to the peptide and having specific immunoreactivity to antibodies to HCV.

3. A peptide composition according to claim 2 further comprising Peptide IIH having an amino acid sequence: 
Ser-Gly-Lys-Pro-Ala-Ile-Ile-Pro-Asp-Arg-Glu-Val-Leu-
Tyr-Arg-Glu-Phe-Asp-Glu-Met-Glu-Glu-Cys-Ser-Gln-His-
Leu-Pro-Tyr-Ile-Glu-Gln-Gly-Met-Met-Leu-Ala-Glu-Gln-
Phe-Lys-Gln-Lys-Ala-Leu-Gly-Leu-X;
(IIH)
 wherein X is -OH or -NH₂, or
an analogue peptide of the above peptide having an amino acid sequence from a strain/isolate of HCV in a region corresponding to the peptide and having specific immunoreactivity to antibodies to HCV.

4. A peptide composition according to claim 2 further comprising pep8 having an amino acid sequence: 
Phe-Thr-Phe-Ser-Pro-Arg-Arg-His-Trp-Thr-Thr-Gln-Gly-
Cys-Asn-Cys-Ser-Ile-Tyr-Pro-Gly-His-Ile-Thr-Gly-His-
Arg-Met-Ala-Trp-Asp-Met-Met-Met-Asn-Trp-Ser-Pro-Thr-
Ala-X;
pep8
 wherein X is -OH or -NH₂, or
an analogue peptide of the above peptide having an amino acid sequence from a strain/isolate of HCV in a region corresponding to the peptide and having specific immunoreactivity to antibodies to HCV.

5. A peptide composition according to claim 2 further comprising pepl2 having an amino acid sequence: 
Lys-Ala-Thr-Cys-Thr-Ala-Asn-His-Asp-Ser-Pro-Asp-Ala-
Glu-Leu-Ile-Glu-Ala-Asn-Leu-Leu-Trp-Arg-Gln-Glu-Met-
Gly-Gly-Asn-Ile-Thr-Arg-Val-Glu-Ser-Glu-Asn-Lys-Val-
Val-Ile-Leu-Asp-Ser-Phe-Asp-Pro-Leu-Val-Ala-Glu-Glu-
Asp-Glu-Arg-X;
pep12
 wherein X is -OH or NH₂; or
an analogue peptide of the above peptide having an amino acid sequence from a strain/isolate of HCV in a region corresponding to the peptide and having specific immunoreactivity to antibodies to HCV.

6. A method of detecting antibodies to HCV or NANBHV by using an effective amount of a peptide composition according to any one of claims 1 to 5 in an immunoassay procedure.

7. A peptide immunogen comprising an octomeric peptide having the structure: [peptide]8Lys4Lys2Lys wherein the peptide is: 
Glu-Ile-Leu-Arg-Lys-Ser-Arg-Arg-Phe-Ala-Gln-Ala-Leu-
Pro-Val-Trp-Ala-Arg-Pro-Asp-Tyr-Asn-Pro-Pro-Leu-Val-
Glu-Thr-Trp-Lys-Lys-Pro-Asp-Tyr-Glu-Pro-Pro-Val-Val-
His-Gly-Cys-Pro-Leu-Pro-Pro-Pro-Lys-Ser-Pro-Pro-Val-
Pro-Pro-Pro-Arg-Lys-Lys-Arg-Thr-X;
Pep11
 wherein X is -OH or -NH₂, or
an analogue peptide of the above peptide having an amino acid sequence from a strain/isolate of HCV in a region corresponding to the peptide and having specific immunoreactivity to antibodies to HCV,
a segment of the above peptide or said analogue peptide having specific immunoreactivity to antibodies to HCV, and
a conjugate of the above peptide, said analogue peptide or said segment with a carrier protein, the conjugate having specific immunoreactivity to antibodies to HCV.

## Patentansprüche

1. Peptidzusammensetzung enthaltend ein Peptid mit einer Aminosäuresequenz: 
Glu-Ile-Leu-Arg-Lys-Ser-Arg-Arg-Phe-Ala-Gln-Ala-Leu-
Pro-Val-Trp-Ala-Arg-Pro-Asp-Tyr-Asn-Pro-Pro-Leu-Val-
Glu-Thr-Trp-Lys-Lys-Pro-Asp-Tyr-Glu-Pro-Pro-Val-Val-
His-Gly-Cys-Pro-Leu-Pro-Pro-Pro-Lys-Ser-Pro-Pro-Val-
Pro-Pro-Pro-Arg-Lys-Lys-Arg-Thr-X;
Pep11
 worin-X -OH oder NH₂ ist, oder
ein analoges Peptid des vorstehenden Peptids mit einer Aminosäuresequenz aus dem Stamm/Isolat von HCV in einem Bereich, der dem Peptid entspricht, und mit spezifischer Immunreaktivität gegenüber Antikörpern gegen HCV,
ein Konjugat des vorstehenden Peptids oder des analogen Peptids mit einem Trägerprotein, wobei das Konjugat eine spezifische Immunreaktivität gegenüber HCV hat, und
ein Polymer des vorstehenden Peptids oder des analogen Peptids mit spezifischer Immunreaktivität gegenüber Antikörpern gegen HCV, wobei das Polymer eine dimere, tetramere, octamere und decahexyle Form des Peptids ist, und das Polymer auf einem verzweigten Poly-L-lysin Harz synthetisiert wird, welches Lys₈; Lys₄; Lys₂; Lys, Lys₄; Lys₂; Lys, Lys₂; Lys, Lys sein kann.

2. Peptidzusammensetzung enthaltend eine Mischung der Peptide VIIIE und Pep 11, worin Peptid VIIIE: 
Ser-Thr-Ile-Pro-Lys-Pro-Gln-Arg-Lys-Thr-Lys-Arg-Asn-
Thr-Asn-Arg-Arg-Pro-Gln-Asp-Val-Lys-Phe-Pro-Gly-Gly-
Gly-Gln-Ile-Val-Gly-Gly-Val-Tyr-Leu-Leu-Pro-Arg-Arg-
Gly-Pro-Arg-Leu-Gly-Val-Arg-Ala-Thr-Arg-Lys-Thr-Ser-
Glu-Arg-Ser-Gln-Pro-Arg-Gly-Arg-Arg-X
(VIIIE)
 und pep11 
Glu-Ile-Leu-Arg-Lys-Ser-Arg-Arg-Phe-Ala-Gln-Ala-Leu-
Pro-Val-Trp-Ala-Arg-Pro-Asp-Tyr-Asn-Pro-Pro-Leu-Val-
Glu-Thr-Trp-Lys-Lys-Pro-Asp-Tyr-Glu-Pro-Pro-Val-Val-
His-Gly-Cys-Pro-Leu-Pro-Pro-Pro-Lys-Ser-Pro-Pro-Val-
Por-Pro-Pro-Arg-Lys-Lys-Arg-Thr-X
Pep11
 sind, worin X -OH oder -NH₂ ist, oder
ein analoges Peptid des vorstehenden Peptids mit einer Aminosäuresequenz von einem Stamm/Isolat von HCV in einem Bereich entsprechend dem Peptid und mit spezifischer Immunreaktivität gegenüber Antikörpern gegen HCV.

3. Peptidzusammensetzung nach Anspruch 2, enthaltend weiter Peptid IIH mit einer Aminosäuresequenz: 
Ser-Gly-Lys-Pro-Ala-Ile-Ile-Pro-Asp-Arg-Glu-Val-Leu-
Tyr-Arg-Glu-Phe-Asp-Glu-Met-Glu-Glu-Cys-Ser-Gln-His-
Leu-Pro-Tyr-Ile-Glu-Gln-Gly-Met-Met-Leu-Ala-Glu-Gln-
Phe-Lys-Gln-Lys-Ala-Leu-Gly-Leu-X,
(IIH)
 worin X -OH oder NH₂ ist, oder
ein analoges Peptid des vorstehenden Peptids mit einer Aminosäuresequenz aus einem Stamm/Isolat von HCV in einem Bereich entsprechend dem Peptid und mit spezifischer Immunreaktivität gegenüber Antikörpern gegen HCV.

4. Peptidzusammensetzung nach Anspruch 2, weiter enthaltend Pep8 mit einer Aminosäuresequenz: 
Phe-Thr-Phe-Ser-Pro-Arg-Arg-His-Trp-Thr-Thr-Gln-Gly-
Cys-Asn-Cys-Ser-Ile-Tyr-Pro-Gly-His-Ile-Thr-Gly-His-
Arg-Met-Ala-Trp-Asp-Met-Met-Met-Asn-Trp-Ser-Pro-Thr-
Ala-X
Pep8
 worin X -OH oder NH₂ ist, oder
ein analoges Peptid des vorstehenden Peptids mit einer Aminosäuresequenz aus einem Stamm/Isolat von HCV in einem Bereich entsprechend dem Peptid und mit spezifischer Immunreaktivität gegenüber Antikörpern gegen HCV ist.

5. Peptidzusammensetzung nach Anspruch 2, weiter enthaltend Pep12 mit einer Aminosäuresequenz: 
Lys-Ala-Thr-Cys-Thr-Ala-Asn-His-Asp-Ser-Pro-Asp-Ala-
Glu-Leu-Ile-Glu-Ala-Asn-Leu-Leu-Trp-Arg-Gln-Glu-Met-
Gly-Gly-Asn-Ile-Thr-Arg-Val-Glu-Ser-Glu-Asn-Lys-Val-
Val-Ile-Leu-Asp-Ser-Phe-Asp-Pro-Leu-Val-Ala-Glu-Glu-
Asp-Glu-Arg-X
Pep12
 worin X -OH oder NH₂ ist, oder
ein analoges Peptid des vorstehenden Peptids mit einer Aminosäuresequenz aus einem Stamm/Isolat von HCV in einem Bereich entsprechend dem Peptid und mit spezifischer Immunreaktivität gegenüber Antikörpern gegen HCV ist.

6. Verfahren zur Bestimmung von Antikörpern gegen HCV oder NANBHV unter Verwendung einer wirksamen Menge einer Peptidzusammensetzung nach einem der Ansprüche 1 bis 5 in einem Immunoassay-Verfahren.

7. Peptidimmunogen enthaltend ein octameres Peptid mit der Struktur: [Peptid]8Lys4Lys2Lys worin das Peptid 
Glu-Ile-Leu-Arg-Lys-Ser-Arg-Arg-Phe-Ala-Gln-Ala-Leu-
Pro-Val-Trp-Ala-Arg-Pro-Asp-Tyr-Asn-Pro-Pro-Leu-Val-
Glu-Thr-Trp-Lys-Lys-Pro-Asp-Tyr-Glu-Pro-Pro-Val-Val-
His-Gly-Cys-Pro-Leu-Pro-Pro-Pro-Lys-Ser-Pro-Pro-Val-
Pro-Pro-Pro-Arg-Lys-Lys-Arg-Thr-X
Pep11
 worin X -OH oder NH₂ ist, oder
ein analoges Peptid des vorstehenden Peptids mit einer Aminosäuresequenz aus einem Stamm/Isolat von HCV in einem Bereich entsprechend dem Peptid und mit spezifischer Immunreaktivität gegenüber Antikörpern gegen HCV,
ein Segment des vorstehenden Peptids oder des analogen Peptids mit spezifischer Immunreaktivität gegenüber Antikörpern gegen HCV, und
ein Konjugat des vorstehenden Peptids, des analogen Peptids oder des Segmentes mit einem Trägerprotein, wobei das Konjugat eine spezifische Immunreaktivität gegenüber Antikörper gegen HCV hat.

## Revendications

1. Composition peptidique comprenant un peptide ayant la séquence d'aminoacides 
Glu-Ile-Leu-Arg-Lys-Ser-Arg-Arg-Phe-Ala-Gln-Ala-Leu-
Pro-Val-Trp-Ala-Arg-Pro-Asp-Tyr-Asn-Pro-Pro-Leu-Val-
Glu-Thr-Trp-Lys-Lys-Pro-Asp-Tyr-Glu-Pro-Pro-Val-Val-
His-Gly-Cys-Pro-Leu-Pro-Pro-Pro-Lys-Ser-Pro-Pro-Val-
Pro-Pro-Pro-Arg-Lys-Lys-Arg-Thr-X;
Pep11
 dans laquelle X est -OH ou -NH₂, ou
un peptide analogue du peptide ci-dessus ayant une séquence d'aminoacides issue d'une souche/isolat de HCV (virus de l'hépatite C) dans une région correspondant au peptide et ayant une immunoréactivité spécifique à l'égard des anticorps anti-HCV,
un conjugué du peptide ci-dessus ou dudit peptide analogue avec une protéine support, le conjugué ayant une immunoréactivité spécifique à l'égard des anticorps anti-HCV, et
un polymère du peptide ci-dessus ou dudit peptide analogue ayant une immunoréactivité spécifique à l'égard des anticorps anti-HCV, ledit polymère représentant une forme dimère, tétramère, octamère et décahexamère du peptide et ledit polymère étant synthétisé sur une résine poly-L-lysine de ramification qui peut être Lys₈; Lys₄; Lys₂; Lys, Lys₄; Lys₂; Lys, Lys₂; Lys, Lys.

2. Composition peptidique comprenant un mélange de peptides VIIIE et pep11, dans lequel le peptide VIIIE est 
Ser-Thr-Ile-Pro-Lys-Pro-Gln-Arg-Lys-Thr-Lys-Arg-Asn-
Thr-Asn-Arg-Arg-Pro-Gln-Asp-Val-Lys-Phe-Pro-Gly-Gly-
Gly-Gln-Ile-Val-Gly-Gly-Val-Tyr-Leu-Leu-Pro-Arg-Arg-
Gly-Pro-Arg-Leu-Gly-Val-Arg-Ala-Thr-Arg-Lys-Thr-Ser-
Glu-Arg-Ser-Gln-Pro-Arg-Gly-Arg-Arg-X;
(VIIIE)
 et pep11 est 
Glu-Ile-Leu-Arg-Lys-Ser-Arg-Arg-Phe-Ala-Gln-Ala-Leu-
Pro-Val-Trp-Ala-Arg-Pro-Asp-Tyr-Asn-Pro-Pro-Leu-Val-
Glu-Thr-Trp-Lys-Lys-Pro-Asp-Tyr-Glu-Pro-Pro-Val-Val-
His-Gly-Cys-Pro-Leu-Pro-Pro-Pro-Lys-Ser-Pro-Pro-Val-
Pro-Pro-Pro-Arg-Lys-Lys-Arg-Thr-X;
Pep11
 dans laquelle X est -OH ou -NH₂, ou
un peptide analogue du peptide ci-dessus ayant une séquence d'aminoacides issue d'une souche/isolat de HCV dans une région correspondant au peptide et ayant une immunoréactivité spécifique à l'égard des anticorps anti-HCV.

3. Composition peptidique selon la revendication 2, comprenant en outre le peptide IIH ayant la séquence d'aminoacides 
Ser-Gly-Lys-Pro-Ala-Ile-Ile-Pro-Asp-Arg-Glu-Val-Leu-
Tyr-Arg-Glu-Phe-Asp-Glu-Met-Glu-Glu-Cys-Ser-Gln-His-
Leu-Pro-Tyr-Ile-Glu-Gln-Gly-Met-Met-Leu-Ala-Glu-Gln-
Phe-Lys-Gln-Lys-Ala-Leu-Gly-Leu-X;
(IIH)
 dans laquelle X est -OH ou -NH₂, ou
un peptide analogue du peptide ci-dessus ayant une séquence d'aminoacides issue d'une souche/isolat de HCV dans une région correspondant au peptide et ayant une immunoréactivité spécifique à l'égard des anticorps anti-HCV.

4. Composition peptidique selon la revendication 2, comprenant en outre le pep8 ayant la séquence d'aminoacides: 
Phe-Thr-Phe-Ser-Pro-Arg-Arg-His-Trp-Thr-Thr-Gln-Gly-
Cys-Asn-Cys-Ser-Ile-Tyr-Pro-Gly-His-Ile-Thr-Gly-His-
Arg-Met-Ala-Trp-Asp-Met-Met-Met-Asn-Trp-Ser-Pro-Thr-
Ala-X;
pep8
 dans laquelle X est -OH ou -NH₂, ou
un peptide analogue du peptide ci-dessus ayant une séquence d'aminoacides issue d'une souche/isolat de HCV dans une région correspondant au peptide et ayant une immunoréactivité spécifique à l'égard des anticorps anti-HCV.

5. Composition peptidique selon la revendication 2, comprenant en outre le pep12 ayant la séquence d'aminoacides: 
Lys-Ala-Thr-Cys-Thr-Ala-Asn-His-Asp-Ser-Pro-Asp-Ala-
Glu-Leu-Ile-Glu-Ala-Asn-Leu-Leu-Trp-Arg-Gln-Glu-Met-
Gly-Gly-Asn-Ile-Thr-Arg-Val-Glu-Ser-Glu-Asn-Lys-Val-
Val-Ile-Leu-Asp-Ser-Phe-Asp-Pro-Leu-Val-Ala-Glu-Glu-
Asp-Glu-Arg-X;
pep12
 dans laquelle X est -OH ou -NH₂, ou
un peptide analogue du peptide ci-dessus ayant une séquence d'aminoacides issue d'une souche/isolat de HCV dans une région correspondant au peptide et ayant une immunoréactivité spécifique à l'égard des anticorps anti-HCV.

6. Procédé de détection d'anticorps anti-HCV ou anti-NANBHV, comprenant l'utilisation d'une quantité efficace d'une composition peptidique selon l'une quelconque des revendications 1 à 5 dans une technique d'analyse immunologique.

7. Immunogène peptidique comprenant un peptide octamère ayant la structure: [peptide]8Lys4Lys2Lys dans laquelle le peptide est: 
Glu-Ile-Leu-Arg-Lys-Ser-Arg-Arg-Phe-Ala-Gln-Ala-Leu-
Pro-Val-Trp-Ala-Arg-Pro-Asp-Tyr-Asn-Pro-Pro-Leu-Val-
Glu-Thr-Trp-Lys-Lys-Pro-Asp-Tyr-Glu-Pro-Pro-Val-Val-
His-Gly-Cys-Pro-Leu-Pro-Pro-Pro-Lys-Ser-Pro-Pro-Val-
Pro-Pro-Pro-Arg-Lys-Lys-Arg-Thr-X;
Pep11
 où X est -OH ou -NH₂, ou
un peptide analogue du peptide ci-dessus ayant une séquence d'aminoacides issue d'une souche/isolat de HCV dans une région correspondant au peptide et ayant une immunoréactivité spécifique à l'égard des anticorps anti-HCV,
un segment du peptide ci-dessus ou dudit peptide analogue ayant une immunoréactivité spécifique à l'égard des anticorps anti-HCV, et
un conjugué du peptide ci-dessus, dudit peptide analogue ou dudit segment avec une protéine support, le conjugué ayant une immunoréactivité spécifique à l'égard des anticorps anti-HCV.
